(19) Europäisches Patentamt
European Patent Office
Office européen des brevets

(11) EP 1 114 704 B1

(12) **EUROPEAN PATENT SPECIFICATION**

(45) Date of publication and mention of the grant of the patent:
**23.11.2005 Bulletin 2005/47**

(21) Application number: **01100855.4**

(22) Date of filing: **09.06.1994**

(51) Int Cl.[7]: **B27K 3/50**, B27K 3/34, C07D 313/04, C07D 309/10, C07D 307/20, C07D 305/08, C07C 69/527, C07C 69/33, C07C 69/30, A01N 33/12, C07C 69/52

(54) **Waterproofing/preservative compositions**

Zusammensetzung zur Wasserdichtung und Konservierung

Compositions impermeabilisantes et conservatrices

(84) Designated Contracting States:
**AT BE CH DE DK ES FR GB GR IE IT LI LU MC NL PT SE**
Designated Extension States:
**SI**

(30) Priority: **09.06.1993 US 74136**
**09.06.1993 US 74312**
**09.06.1993 US 74313**
**09.06.1993 US 74314**

(43) Date of publication of application:
**11.07.2001 Bulletin 2001/28**

(62) Document number(s) of the earlier application(s) in accordance with Art. 76 EPC:
**94920194.1 / 0 702 517**

(73) Proprietor: **LONZA INC.**
**Fair Lawn New Jersey 07410 (US)**

(72) Inventor: **Walker,Leigh**
**Macungie, PA 18062 (US)**

(74) Representative: **Riegler, Norbert Hermann**
**Lonza AG**
**Patentabteilung**
**Postfach**
**4002 Basel (CH)**

(56) References cited:
**EP-A- 0 107 479**
**EP-A- 0 340 816**
**WO-A-87/06177**
**GB-A- 1 540 386**
**US-A- 4 732 817**

- **DATABASE WPI Section Ch, Week 199140 Derwent Publications Ltd., London, GB; Class A97, AN 1985-220779 XP002168898 & JP 03 058325 B (DAINIPPON INK & CHEM KK) , 5 September 1991 (1991-09-05)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to waterproofing wood preservation compositions. Polyhydroxyl or polyether hydroxyl esters of fatty acids in combination with quaternary ammonium compositions and a solvent are useful as waterproofing wood preservation compositions. Preferred quaternary ammonium compositions include $C_1$-$C_{20}$ alkyl or aryl-substituted alkyl, $C_8$-$C_{20}$ alkyl quaternary ammonium chlorides, hydroxides, carbonates, carboxylates, or borates.

Quaternary ammonium compounds (quats), and particularly didecyldimethylammonium chloride (DDAC) are commonly used as wood preservatives because they possess resistance properties to fungi and termites, to loss of strength, and to electrical sensitivity similar to those of commonly used acidic copper/chromium/arsenic solution (CCA) or ammoniacal copper and arsenic salt solution preservatives. See Proc of the Am. Wood Pres. Assoc., 80:191-210 (1984). Although chloride quats do not include potentially dangerous heavy metals, didecyldimethylammonium chloride leaches rapidly in soil (Nicholas et al., Forest. Prod. J., 41:41 (1991), and therefore, does require coupling with copper salt.

Findlay et al., U.S. Patent No. 4,929,454, disclose a method of preserving wood by impregnation with a quaternary ammonium compound and at least one of zinc and copper, wherein the quat anion is chosen from the group consisting of hydroxide, chloride, bromide, nitrate, bisulfate, acetate, bicarbonate, and carbonate, formate, borate and anions of fatty acids. These quats have distinct environmental and safety advantages over commonly used acidic copper/chromium/arsenic solution (CCA) or ammoniacal copper and arsenic salt solution preservatives in that potentially dangerous heavy metals are not included. The Findlay et al. quats require copper or zinc in order to render them relatively insoluble and to prevent them from leaching out of a treated substrate. The use of copper or zinc in the above formulations may yet raise environmental and corrosion concerns.

Additionally, didecyldimethylammonium chloride tends to absorb preferentially to the surface of the wood and does not uniformly treat the whole substrate. Finally, DDAC treated wood shows a surface erosion or ages upon exposure to light. See Preston et al., Proc. Am. Wood Pres. Assoc., 83:331 (1987).

The biocidal activities of various chloride quats against bacteria, fungi, and algae are tabulated in Cationic Surfactants, B. Jungerman Ed., pp. 56-57, Marcel Dekker, Inc., 1969. Nicholas, "Interaction of Preservatives with Wood," Chemistry of Solid Wood, Advances in Chemistry Series #207, Powell ed., (A.C.S. 1984), notes that didecyldimethylammonium compounds and particularly DDAC are potential biocides. Preston, J.A.O.C.S. 60:567 (1983), concurs and suggests that maximum fungitoxicity is exhibited with dialkyldimethyl compounds having $C_{10}$-$C_{12}$ alkyl groups. Butcher et al., Chem.Abstracts No. 91:152627b, suggest that the presence of an acid or a base can affect the activity of didecyldimethylammonium quats.

Didecyldimethylammonium acetate was used as a phase transfer catalyst for an oxidation in Chem Abstracts No. 97:9175. A wood preservative was prepared by autoclaving didecylmethylamine with gluconic acid and ethylene oxide in isopropanol to yield $[(C_{10}H_{21})_2CH_3N((CH_2)_2O)_nH]^+$ gluconate in Chem. Abstracts No. 109:124403x, while disinfectant solutions were prepared by exchanging a benzylammonium chloride with chlorhexidine gluconate in Chem. Abstracts No. 103:109954f.

Microbiocidal compositions which include quaternary ammonium compounds of the formula $R^1N^+R^2R^3R^4\ X^-$, wherein at least one of $R^1$, $R^2$, or $R^3$ is a $C_8$-$C_{30}$ alkyl or alkenyl group and the remainder of $R^1$, $R^2$ or $R^3$ is methyl, ethyl, $CH_2Ph$ or 4-pyridylmethyl; $R^4$ is methyl or ethyl; and X is an anion of an acid having a $C_7$ or greater hydrophobic group, were disclosed in Chem. Abstracts Nos. 113:154360f and 113:153776j. Chem.Abstracts No. 112:79768u discloses compounds of the formula $R^1R^2R^3R^4N^+X^-$, wherein $R^1$, $R^2$, and $R^3$ are methyl, ethyl, benzyl, 4-pyridinomethyl and at least one is $C_8$-$C_{30}$ alkyl or alkenyl; $R^4$ is methyl or ethyl; and X is a counter anion of acids having $C_7$ or greater hydrophobic groups. Dimethyldidecylammonium dodecylbenzenesulfonate was demonstrated to impart long term rot resistance to wood without causing rust, while the chloride salts of similar compounds were demonstrated to cause rust.

Patton et al., U.S. Patent No. 5,004,760, disclose polymeric foams incorporating various dialkyldimethylammonium carboxylates such as didecyldimethylammonium poly(ethylene/acetate) and the like.

Quaternary ammonium compounds (quats) are typically prepared by the reaction:

$$R^1R^2R^3N + R^4X \rightarrow R^1R^2R^3R^4NX \qquad (II)$$

wherein X is a halogen, a sulfate, a sulfo compound, or the like. When at least one of $R^1$, $R^2$, $R^3$, or $R^4$ is $C_{12}$ or longer, the product is an inert soap. Many of the inert soaps have biocidal activity against bacteria, fungi, algae, and related organisms.

General quaternary ammonium compounds with a sulfo group are easily prepared either by the reaction of a sulfonate compound with a tertiary amine (III) or by a double exchange (IV).

$$R_3N + RSO_3CH_3 \rightarrow R_3NCH_3^+ RSO_3^- \qquad (III)$$

$$R_3N^+ CH_3 Cl^- + RSO_3^- Na^+ \rightarrow R_3NCH_3^+ RSO_3^- + NaCl \qquad (IV)$$

If trimethylamine is heated with carbon dioxide and methanol above 200°C and at 85 to 95 atmospheres, the carbonate quat, bis-tetramethylammonium carbonate, is prepared. Industrial Organic Nitrogen Compounds, Astle Ed. p.66, Reinhold Inc, 1961. However, this reaction is limited to the methyl compound because higher homologs decompose to olefins by the Hofman elimination reaction. See, Organic Reactions, 11, Chptr. 5, 377, Krieger Publishing Co., 1975.

Chem. Abstracts 110:212114 (1989) suggests that dimethyl carbonate will react with triethylamine in methanol in twelve hours at 115°C and under pressure to yield a methyl carbonate ester quat.

Chem.Abstracts 114:24824 (1991) discloses that 6-hydroxyhexyldimethylamine reacts with dimethyl carbonate to yield a carbonate ester quat.

Quaternary ammonium hydroxides (hydroxy quats), an intermediate in the reaction scheme, are currently prepared by the reaction of quaternary ammonium iodide with silver hydroxide (V) .

$$RN^+ (CH_3)_3) I^- + AgOH \rightarrow RN^+(CH_3)_3OH^- + AgI \qquad (V)$$

However, this reaction is costly, and it is difficult to recover the silver reagent. See, Organic Reactions, 11:Chptr 5, pp. 376-377, Krieger Publishing Co., 1975.

In an olefin synthesis, it has been suggested to treat a quaternary salt with aqueous sodium or potassium hydroxide followed by pyrolysis in order to form the hydroxy quat and then to decompose the hydroxy quat directly. However, in this method the hydroxy quat is not isolated and the conditions for its preparation are undesirable. See, Organic Reactions, 11:Chptr 5, pp. 376-377, Krieger Publishing Co., 1975.

Talmon et al., Science, 221, 1047 (1983), have used an ion exchange resin to convert didecyldimethylammonium bromide to didecyldimethylammonium hydroxide. However, 50 ml of ion exchange resin and two treatment steps were required to convert 3 g of quaternary ammonium chloride to the corresponding hydroxide. Talmon et al. state that the hydroxy quat can be reacted with acids to make quats with different anions, and they have prepared didodecyldimethylammonium (DDDA) acetate, DDDA-formate, DDDA-propionate, DDDA-butyrate, DDDA-oxalate, DDDA-acrylate, DDDA-tartrate, DDDA-benzoate, and DDDA-octanoate. See also, Organic Synthesis, Collective Volume VI, 552, John Wiley Inc., 1988; Brady et al., J. Am. Chem. Soc., 106:4280-4282, 1984; Brady et al., J.Phys. Chem., 90:9, 1853-1859, 1986; Miller et al., J. Phys. Chem, 91:1, 323-325, 1989; Radlinske et al., Colloids and Surfaces, 46:213-230, 1990.

Distearyldimethylammonium gluconate was prepared via ion exchange and subsequent reaction with an organic acid in Chem. Abstracts No. 75:11917OU. Miller et al., Langmuir, 4:1363 (1988) prepared ditetradecyldimethylammonium acetate by ion exchange from a bromide.

Alternatively, quaternary ammonium hydroxide compositions have been prepared by treating a haloquat in an electrochemical cell with special cation exchange diaphragms between the cells. The hydroxy quat collects at one electrode, and the halide collects at the other. Hydroxy quats, $R^1R^2R^3R^4N^+OH^-$, wherein the R groups were $C_1$-$C_4$, were treated with carboxylic acids to make asymmetric quats that were used as capacitor driving electrolytes. See, Japanese Patent Publication No. 02-106,915 and Awata et al., Chemistry, Letters, 371 (1985). Awata et al. placed carboxylic acids in the cathode cell to react with tetraethylammonium hydroxide as it was formed.

Japanese Patent Publication No. 01-172,363 discloses the preparation of relatively low yields of tetraethylammonium hydroxide by reacting triethylamine with diethyl sulfate, heating the resultant quat with sulfuric acid to yield the sulfate quat, and reacting the sulfate quat with barium hydroxide to yield the short chain quat, tetraethylammonium hydroxide, and barium sulfate.

Di $C_8$-$C_{12}$ alkyl quaternary ammonium hydroxides prepared by ion exchange were used as strong bases to digest animal tissue by Bush et al., French Patent Publication No. 1,518,427.

Akzo discloses that the addition of a metallic hydroxide to a quaternary ammonium chloride such as didecyldimethylammonium chloride, in an aqueous medium, results in an equilibrium mixture of quaternary ammonium chloride and quaternary ammonium hydroxide. This reaction can be driven to the right by the use of isopropanol as a solvent.

Akzo further discloses that the addition of a soap to a quaternary ammonium chloride yields a quaternary ammonium carboxylate.

Jordan et al., U.S. Patent No. 3,281,458, disclose the preparation of dioctadecyldimethylammonium humate,

ditallowdimethylammonium humate, dipentadecyldimethylammonium humate, and didodecyldimethylammonium humate by reacting humic acid, lignite, aqueous sodium hydroxide and a chloride quat.

WO 87/06177 A1 discloses ammoniacal solutions containing rosin, a preservative metal and a nonionic tenside or a quaternary ammonium compound. The solutions are suitable for use as treatment agents for wood and wood based products to render them water-repellent and impart a fungicidal effect. Among the nonionic tensides disclosed are fatty acid esters of polyvalent alcohols, such as glycol, glycerol or sorbitan.

US 4,732,817 discloses an organic solvent-free process for treating pressure permeable wood with solutions containing tannin extracts and a fixative material which is preferably a nonionic surface active agent, optionally in combination with cationic surfactants.

Finally, Nakama et al., J.A.C.O.S., 67:717 (1990) report the interaction between anionic and cationic surfactant and particularly sodium laurate and stearyltrimethylammonium chloride, while Linderborg, U.S. Patent No. 4,585,795, disclose the use of synergistic mixtures of the alkali metal salt of certain biocidal organic acids, quaternary ammonium chlorides, and alkyl-pyridinium chlorides as control agents for short-term protection of timber against sapstain fungi and mildew.

Consequently, efforts have been directed to develop a safe, efficient and expedient method to prepare quaternary ammonium compounds that do not require potentially hazardous metal additives to treat wooden substrates effectively.

It has now been discovered that useful $C_1$-$C_{20}$ alkyl or aryl-substituted alkyl, $C_8$-$C_{20}$ alkyl quaternary ammonium hydroxides can be prepared from specific quaternary chlorides and a metal hydroxide.

It has further been discovered that useful $C_1$-$C_{20}$ alkyl or aryl-substituted alkyl, $C_8$-$C_{20}$ alkyl quaternary ammonium carbonates can be prepared, particularly by indirect synthesis from $C_1$-$C_{20}$ alkyl or aryl-substituted alkyl, $C_8$-$C_{20}$ alkyl quaternary ammonium chlorides, through $C_1$-$C_{20}$ alkyl or aryl-substituted alkyl, $C_8$-$C_{20}$ alkyl quaternary ammonium hydroxide intermediates.

$C_1$-$C_{20}$ alkyl or aryl-substituted alkyl, $C_8$-$C_{20}$ alkyl carboxylate quats, and particularly the di $C_8$-$C_{12}$ alkyl carboxylate quats above, can be prepared by various methods from $C_1$-$C_{20}$ alkyl or aryl-substituted alkyl, $C_8$-$C_{20}$ alkyl quaternary ammonium chloride (chloride quat(s)) starting materials, including by indirect synthesis through $C_1$-$C_{20}$ alkyl or aryl-substituted alkyl, $C_8$-$C_{20}$ alkyl quaternary ammonium hydroxide and $C_1$-$C_{20}$ alkyl or aryl-substituted alkyl, $C_8$-$C_{20}$ alkyl quaternary ammonium carbonate intermediates or by direct synthesis. $C_1$-$C_{20}$ alkyl or aryl-substituted alkyl, $C_8$-$C_{20}$ alkyl borate quats, and particularly the di $C_8$-$C_{12}$ alkyl borate quats above can be prepared by various methods from $C_1$-$C_{20}$ alkyl or aryl-substituted alkyl, $C_8$-$C_{20}$ alkyl quaternary ammonium hydroxide starting materials, which may be prepared as above from the corresponding chloride quats.

Typically, quaternary ammonium compounds migrate or leach from wood under wet conditions, however. Common waterproofing compositions have not proven compatible with the quaternary ammonium compounds typically used in the industry, and therefore, they are not commonly used to hinder the leaching of these quats.

Typical waterproofers are waxes, lower molecular weight polyolefins, or dispersions or solutions thereof in hydrocarbon solvents. However, quaternary compositions, including those useful in the present invention, typically are water soluble. Generally, they are not soluble in these typical waterproofer solvent systems and are not compatible with emulsified or dispersed waterproofers.

It has now been discovered that $C_1$-$C_{20}$ alkyl or aryl-substituted alkyl, $C_8$-$C_{20}$ alkyl, and particularly di $C_8$-$C_{12}$ alkyl, quaternary ammonium hydroxides, carbonates, carboxylates, and borates including those prepared by the methods described herein, are compatible with polyhydroxyl or polyetherhydroxyl esters of fatty acids. Waterproofing and wood preservative systems prepared from the waterproofers and quats described herein exhibit enhanced resistance to leaching and meet waterproofing standards for heavy duty, ground, or millwork applications.

DESCRIPTION OF THE DRAWINGS

Figure 1A is a graphic comparison of leaching of waterproofer containing wood preservative systems according to the present invention and wood preservative systems without waterproofer.

Figure 1B is an enlarged section of Figure 1A.

A high yield method for the preparation of $C_1$-$C_{20}$ alkyl or aryl-substituted alkyl, $C_8$-$C_{20}$ alkyl quaternary ammonium hydroxide, and preferably di $C_8$-$C_{12}$ alkyl quaternary ammonium hydroxide, which includes the selection of particular solvents, has been discovered. Product yield can be further enhanced by adjustment of the amounts of the reactants.

The method comprises reacting two reactants, a $C_1$-$C_{20}$ alkyl or aryl-substituted alkyl, $C_8$-$C_{20}$ alkyl quaternary ammonium chloride, preferably a di $C_8$-$C_{12}$ alkyl quaternary ammonium chloride, and a metal hydroxide, in a solvent

comprising a $C_1$-$C_4$ normal alcohol. The amount of metal hydroxide reactant is that amount sufficient to yield the $C_1$-$C_{20}$ alkyl or aryl-substituted alkyl, $C_8$-$C_{20}$ alkyl quaternary ammonium hydroxide and a metal chloride. Preferably, this amount is at least a stoichiometric amount.

Quaternary ammonium carbonates having the formula

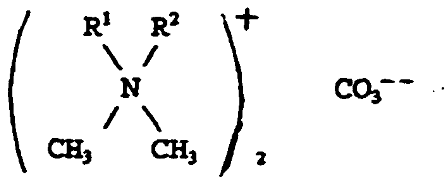

(IX)

wherein $R^1$ is a $C_1$-$C_{20}$ alkyl or aryl-substituted alkyl group and $R^2$ is a $C_8$-$C_{20}$ alkyl group, and preferably wherein $R^1$ is the same as $R^2$ and $R^1$ is a $C_8$-$C_{12}$ alkyl group, as well as compositions further comprising the corresponding quaternary ammonium bicarbonate

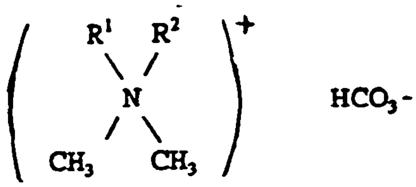

(X)

wherein $R^1$ is the same or a different $C_1$-$C_{20}$ alkyl or aryl-substituted alkyl group as above and $R^2$ is the same or a different $C_8$-$C_{20}$ alkyl group as above, but preferably wherein $R_1$ is the same as $R^2$ and $R^1$ is a $C_8$-$C_{12}$ alkyl group; and/or the corresponding quaternary ammonium metal carbonate

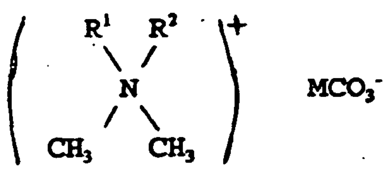

(XI)

wherein $R^1$ is the same or a different $C_1$- $C_{20}$ alkyl or aryl-substituted alkyl group and $R^2$ is a $C_8$-$C_{20}$ alkyl group; but preferably wherein $R^1$ is the same as $R^2$ and $R^1$ is a $C_8$-$C_{12}$ alkyl group and M is a mono-, bi-, or trivalent metal, preferably a monovalent metal, and most preferably an alkali metal, are prepared by reacting two reactants, (a) $C_1$-$C_{20}$ alkyl or aryl-substituted alkyl, $C_8$-$C_{20}$ alkyl quaternary ammonium chloride and preferably a di $C_8$-$C_{12}$ alkyl quaternary ammonium chloride and (b) a metal hydroxide, in a solvent comprising a $C_1$-$C_4$ normal alcohol. The amount of metal hydroxide reactant is that amount sufficient to yield the corresponding $C_1$-$C_{20}$ alkyl or aryl-substituted, $C_8$-$C_{20}$ alkyl quaternary ammonium hydroxide, and preferably the corresponding di $C_8$-$C_{12}$ alkyl quaternary ammonium hydroxide, a metal chloride, and optionally unreacted metal hydroxide. The resultant quaternary ammonium hydroxide and any unreacted metal hydroxide are then reacted with carbon dioxide to yield the corresponding quaternary ammonium carbonate, optionally the corresponding quaternary ammonium bicarbonate, and optionally the corresponding quaternary ammonium metal carbonate, or a combination of any of the foregoing, and optionally metal carbonate.

Di $C_8$-$C_{12}$ alkyl quaternary ammonium carboxylate having the formula

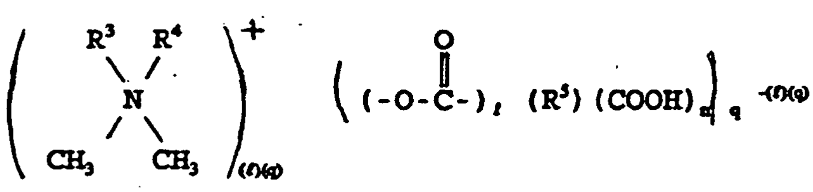

(XII)

wherein $R^3$ is a $C_1$-$C_{20}$ alkyl or aryl-substituted alkyl group; $R^4$ is a $C_8$-$C_{20}$ alkyl group, but preferably $R^3$ and $R^4$ are the same $C_8$-$C_{12}$ alkyl group; $R^5$ is a substituted or unsubstituted, interrupted or uninterrupted $C_1$-$C_{100}$ group; $\ell$ and q independently are 1, 2 or 3 and ($\ell$) (q) is 1, 2, or 3; and n is 0 or an integer from 1 to 50 are preferably prepared by indirect or direct synthesis. The indirect synthesis comprises reacting two reactants, a $C_1$-$C_{20}$ alkyl or aryl-substituted alkyl, $C_8$-$C_{20}$ alkyl, and preferably a di $C_8$-$C_{12}$ alkyl, quaternary ammonium chloride and a metal hydroxide, in a solvent comprising a $C_1$-$C_4$ normal alcohol. The amount of metal hydroxide reactant is that amount sufficient to yield a $C_1$-$C_{20}$ alkyl or aryl-substituted alkyl, $C_8$-$C_{20}$ alkyl quaternary ammonium hydroxide (hydroxide quat (s)) ; and preferably a di $C_8$-$C_{12}$ alkyl quaternary ammonium hydroxide; a metal chloride; and optionally unreacted metal hydroxide. The resultant quaternary ammonium hydroxide and any unreacted metal hydroxide are then reacted with carbon dioxide to yield a $C_1$-$C_{20}$ alkyl or aryl-substituted alkyl, $C_8$-$C_{20}$ alkyl quaternary ammonium carbonate, and preferably a di $C_8$-$C_{12}$ alkyl quaternary ammonium carbonate; and optionally a metal carbonate. The resultant quaternary ammonium carbonate is reacted with carboxylic acid(s) having the formula

$$((R^5)\ (COOH)_{(\ell+n)})_q \qquad (XIV)$$

wherein $R^5$, $\ell$, n, and q are defined as above, to yield the carboxylate quat.

Alternatively, the direct synthesis method comprises reacting a $C_1$-$C_{20}$ alkyl or aryl-substituted alkyl, $C_8$-$C_{20}$ alkyl quaternary ammonium chloride, and preferably a di $C_8$-$C_{12}$ alkyl quaternary ammonium chloride, with at least one metal carboxylate having the formula

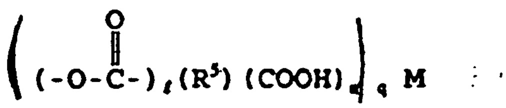

(XV)

wherein $R^5$ and n are as defined above; M is a mono- di-, or tri-valent metal; $\ell$ and q independently are 1, 2, or 3; and ($\ell$) (q) is 1 if M is mono-valent, 2 if M is di-valent, and 3 if M is tri-valent.

Borate quats are preferably prepared by a hydroxy/borate synthesis wherein a hydroxide quat as described above is reacted with boric acid.

According to the present invention, the use of waterproofer compositions for the treatment and preservation of wood is provided.

The waterproofer compositions have the formula

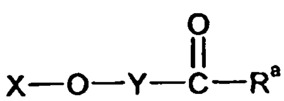

wherein:

X is hydrogen or

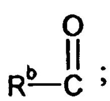

Y is

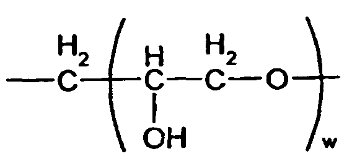

,

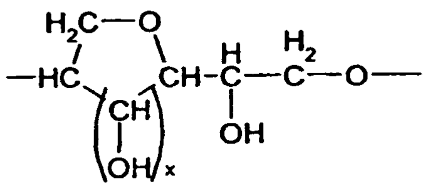

or an enantiomer thereof, or

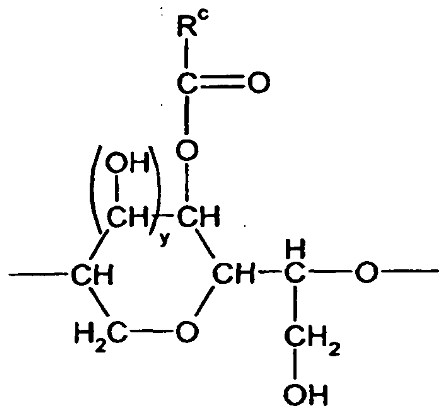

or an enantiomer thereof;
$R^a$, $R^b$ and $R^c$ independently are a saturated or unsaturated $C_9$-$C_{50}$ group;
w is an integer from 1 to 10; and
x is 1 and y is 0;
and the compositions contain a biocidal effective amount of at least one $C_1$-$C_{20}$ alkyl or aryl-substituted alkyl, $C_8$-$C_{20}$ alkyl quaternary ammonium composition and a solvent, and are metal-free.

The polyhydroxyl or polyether hydroxyl fatty acid ester waterproofers are soluble in both aqueous and organic solvent systems. Furthermore, they render the water-soluble quats useful in aqueous or organic systems as well. This occurs despite the fact that these quats alone, i.e. without the present waterproofers, are relatively insoluble in organic solvents, emulsions or dispersions., i.e. they are not generally useful in preserving wood when in an organic solvent.

Saturated $C_9$-$C_{50}$ groups include $C_9$-$C_{50}$ straight chain, branched, or cycloalkyl groups. Unsaturated $C_9$-$C_{50}$ groups include those groups having one or more double or triple bonds or combinations thereof including acyclic groups (including straight chain or branched), cyclic groups, or combinations thereof unsaturated groups. In combinations, unsaturation may occur in the cyclic portion, the acyclic portion, or both.

In a preferred embodiment, X is hydrogen; Y is

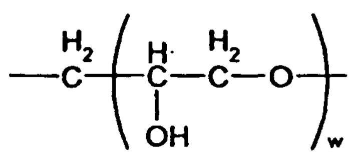

$R^a$ is a $C_{11}$-$C_{17}$ alkyl group, and w is 1.

Special mention is made of

(A) glycerol monostearate wherein Y is

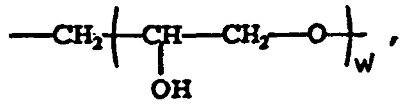

X is hydrogen, $R^a$ is a $C_{17}$ alkyl group, and W is 1;
(B) glycerol monolaurate wherein Y is

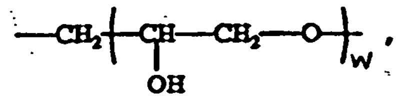

X is hydrogen, $R^a$ is a $C_{11}$ alkyl group, and w is 1; and
(C) cyclic polyhydroxides such as

(1) sorbitan monostearate wherein Y is

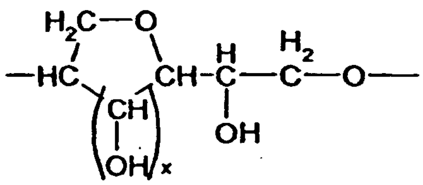

or an enantiomer thereof; is hydrogen, $R^a$ is a $C_{17}$ alkyl group, and x is 1, or
(2) sorbitan tristearate wherein Y is

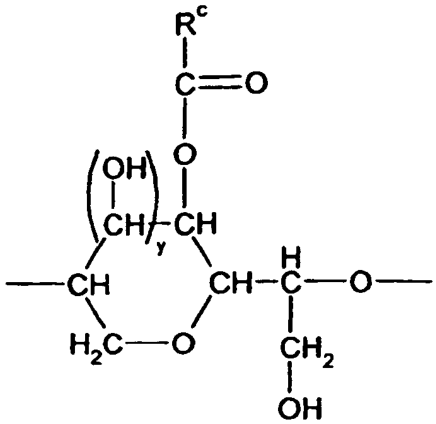

or an enantiomer thereof;

X is

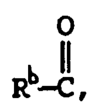

$R^a$, $R^b$, and $R^c$ each are a $C_{17}$ alkyl group, and y is 0.

These waterproofers hinder migration of the quat molecules from a substrate under wet conditions. Furthermore, where surface corrosion problems are related to the water holding properties of the quat, the waterproofer displaces or prevents the entry of water.

The waterproofer, preservative systems used in the present invention include a suitable solvent including aqueous and non-aqueous solvents. Preferably, the solvent is an aqueous solvent including, but not limited to, water, aqueous alcohol, ammonia water, aqueous acetic acid, and the like, or a combination of any of the foregoing. Organic solvents may also be used. These include, but are not limited to, mineral spirits-based solvents and the like.

The amount of waterproofer used in the waterproofer, wood preservative systems of the present invention is

a waterproofing and compatibilizing enhancing amount, i.e. that amount effective to impart or to increase the water resistance, leaching resistance, and/or dimensional stability of the waterproofer, wood preservative system and/or the quat and to enhance the compatibility of the quats of the present invention with a solvent.

The amount of quaternary ammonium composition(s) is a biocidal effective amount, i.e. that amount effective to inhibit the growth of or to kill one or more organism that causes wood rot, to inhibit sap stain, or a combination thereof. Such organisms include, but are not limited to, Trametes viride or Trametes versicolor, which cause a white rot; Gloeophyllum trabeum, which causes a brown rot; and Aspergillus niger, which causes sap stain/mold.

Typically, a waterproofer, wood preservative system will comprise from about 0.1 to about 15 parts by weight of waterproofer(s), from about 0.1 to about 10 parts by weight of quat (s), and from about 99.8 to about 75 parts by weight of solvent based upon 100 parts by weight of quat, waterproofer, and solvent combined. Preferably, the waterproofer, wood preservative systems of the present invention will comprise from about 0.5 to about 6 parts by weight of quat (s) from about 0.5 to about 8.5 parts by weight of waterproofer(s), and from about 96 to about 85.5 parts by weight of solvent on the same basis.

The components of the waterproofer, wood preservative systems used in the present invention are mixed by conventional means known to those skilled in the art preferably to form an emulsion. Preferably, the waterproofer and the quat are melted together. The melt can then be stirred, and warm water (about 40 to 50°C) added with stirring to yield an emulsion or solution. Emulsions prepared in this manner may be stable for periods of at least one year.

Although other conventional additives including, but not limited to, emulsifiers may be added as required for application to different substrates and for different uses as known to those of ordinary skill in the art, metal stabilizers are not present. Accordingly, wood substrates, such as lumber, timber, or the like, can be treated with these systems.

Treatment of the substrate is accomplished by any means known to those of ordinary skill in the art including, but not limited to, dipping, soaking, brushing, pressure treating, or the like. The length of treatment required will vary according to treatment conditions, the selection of which are known to those skilled in the art.

The waterproofer, wood preservative systems used in the present invention display greater resistance to leaching and greater waterproofing properties, as indicated by swell index, than wood preservatives currently used in the industry. Resistance to leaching is defined as retention of a biocidal effective amount, and preferably at least about 2% by weight, of quat in the substrate over a prolonged period of at least about 100 hours and preferably about 350 hours. Although any positive swell index indicates some waterproofing ability, a swell index of greater than about 50 indicates notable waterproofing properties.

In a preferred embodiment, the $C_1$-$C_{20}$ alkyl or aryl-substituted alkyl, $C_8$-$C_{20}$ alkyl quaternary ammonium compositions are selected from the group consisting of quaternary ammonium chlorides, hydroxides, carbonates, carboxylates, borates, or any combination thereof.

Preferred hydroxide quats are di $C_8$-$C_{12}$ alkyl quaternary ammonium hydroxides. Preferred carbonate quats are those having the formula

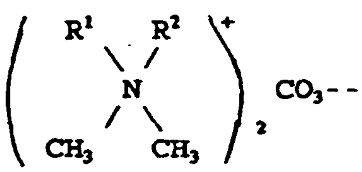

(XIX)

wherein $R^1$ is a $C_1$-$C_{20}$ alkyl or aryl-substituted alkyl group and $R^2$ is a $C_8$-$C_{12}$ alkyl group, or mixtures of

(a) at least one di $C_8$-$C_{12}$ alkyl quaternary ammonium carbonate having the formula

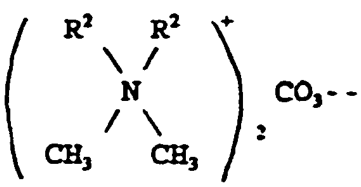

(XX)

wherein $R^2$ is a $C_8$-$C_{12}$ alkyl group; and

(b)

(1) at least one di $C_8$-$C_{12}$ alkyl quaternary ammonium bicarbonate having the formula

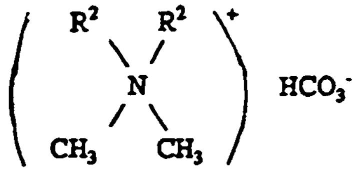

(XXI)

wherein $R^2$ is the same or a different $C_8$-$C_{12}$ alkyl group as in (a) ; or
(2) at least one di $C_8$-$C_{12}$ alkyl quaternary ammonium metal carbonate having the formula

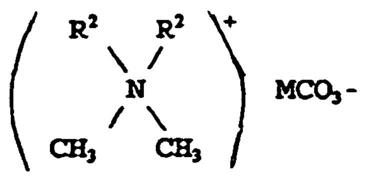

(XXII)

wherein $R^2$ is the same or a different $C_8$-$C_{12}$ alkyl group as in (a) or (b) and M is a non-coupler metal, or
(3) a combination of (b) (1) and (b) (2). Preferred quaternary ammonium carboxylates are those having the formula

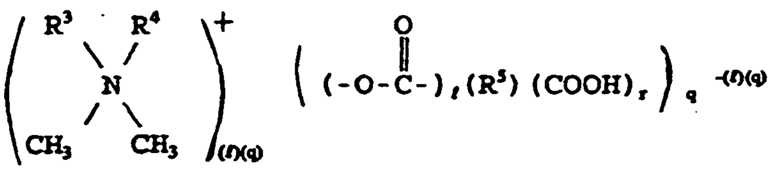

(XXIII)

wherein $R^3$ is a $C_1$-$C_{20}$ alkyl or aryl-substituted alkyl group; $R^4$ is a $C_8$-$C_{20}$ alkyl group; $R^5$ is a substituted or unsubstituted, interrupted or uninterrupted, $C_1$-$C_{100}$ group; $\ell$ and q independently are 1, 2, or 3, and $(\ell)(q)$ is 1, 2, or 3; and r is 0 or an integer from 1 to 50. Preferred quaternary ammonium borates are those having the formula

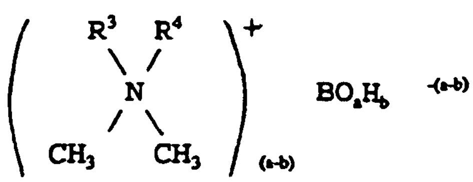

wherein $R^3$ is a $C_1$-$C_{20}$ alkyl or aryl-substituted alkyl group; $R^4$ is a $C_8$-$C_{20}$ alkyl group, a is 2 or 3, but when a is 2, b is 0 or 1 and when a is 3, b is 0, 1 or 2.

Quaternary Ammonium Hydroxides

Although any quaternary ammonium hydroxides are suitable for use in the present invention, quaternary ammonium hydroxides (hydroxy quats) having the formula

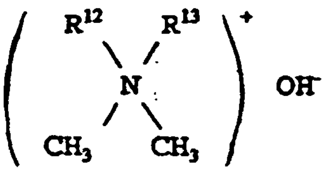

(XVI)

wherein $R^{12}$ is a $C_1$-$C_{20}$ alkyl or aryl-substituted alkyl group, $R^{13}$ is a $C_8$-$C_{20}$ alkyl group, and preferably $R^{12}$ is the same as $R^{13}$ and $R^{12}$ is a $C_8$-$C_{12}$ alkyl group, are preferred.

Special mention is made of hydroxy quats wherein $R^{12}$ is a methyl, $C_8$ alkyl, $C_9$ isoalkyl, $C_{10}$ alkyl, $C_{12}$ alkyl, $C_{14}$ alkyl, $C_{16}$ alkyl, or benzyl group; and $R^{13}$ is a $C_{10}$ alkyl, $C_{12}$, $C_{14}$ alkyl or $C_{16}$ alkyl group. Most preferred hydroxy quats are didecyldimethylammonium hydroxide wherein $R^{12}$ and $R^{13}$ are a $C_{10}$ alkyl group and most preferably an n-$C_{10}$ group.

Didecyldimethylammonium hydroxide, when observed in a 70 to 80 percent by weight solution in a 50 percent by weight alcohol/50 percent by weight water solvent, is a yellow/orange liquid. This formulation has a flash point of about 56.7 °C (134 °F), and it is a highly alkaline material that reacts with the phenolic OH of lignin.

Quaternary ammonium hydroxides useful in the present invention are preferably prepared according to the reaction illustrated below in scheme XXV.

The method provides increased yields of $C_1$-$C_{20}$ alkyl or aryl-substituted alkyl, $C_8$-$C_{20}$ alkyl quaternary ammonium hydroxide, and preferably di $C_8$- $C_{12}$ alkyl quaternary ammonium hydroxide, when compared with conventional production methods. Although it was previously believed that the reaction of the chloride quat salt with a metal hydroxide to yield quaternary ammonium hydroxide and metal chloride was an equilibrium reaction or could be driven to the right by the use of branched solvents, it has now been discovered that by selection of the proper reactants, reaction medium, and/or reaction conditions (including reactant amounts), the reaction can be driven well past equilibrium to yield unprecedented greater amounts of $C_1$-$C_{20}$ alkyl or aryl-substituted alkyl, $C_8$-$C_{20}$ alkyl quaternary ammonium hydroxide.

Although the present method can be used to prepare a variety of $C_1$-$C_{20}$ alkyl or aryl-substituted alkyl, $C_8$-$C_{20}$ alkyl quaternary ammonium hydroxide compounds, the preferred reaction product quat is a di $C_8$-$C_{12}$ alkyl quaternary ammonium hydroxide compound. Most preferred hydroxy quats are di n-$C_8$-$C_{12}$ alkyl quaternary ammonium hydroxide, didecyldimethylammonium hydroxide, and di-n-decyldimethylammonium hydroxide.

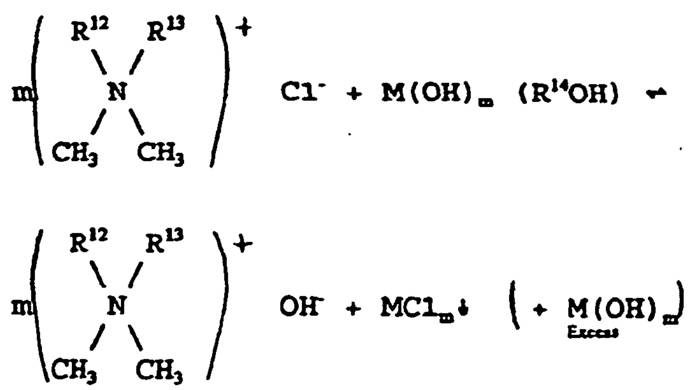

(XXV)

wherein $R^{12}$ is a $C_1$-$C_{20}$ alkyl or aryl-substituted alkyl group; $R^{13}$ is a $C_8$-$C_{20}$ alkyl group; $R^{14}$ is a straight chain $C_1$-$C_4$ alkyl group; M is a mono-, di-, or trivalent metal; and m is one if M is monovalent, two if M is divalent, and three if M is trivalent. Preferably $R^{12}$ is the same as $R^{13}$, i.e. a $C_8$-$C_{12}$ alkyl group.

Many $C_1$-$C_{20}$ alkyl or aryl-substituted alkyl, $C_8$-$C_{20}$ alkyl quaternary ammonium chlorides are suitable reactants, but di $C_8$-$C_{12}$ alkyl quaternary ammonium chloride is preferred, and didecyldimethylammonium chloride, and particularly, di-n-decyldimethylammonium chloride is most preferred. The selections of the $R^{12}$ and $R^{13}$ substituents of the chloride quat reactant are determinative of the hydroxy quat product.

Special mention is also made of processes wherein $R^{12}$ is a methyl, butyl, $C_8$ alkyl, $C_9$ isoalkyl, $C_{10}$ alkyl, $C_{12}$ alkyl, $C_{14}$ alkyl or benzyl group; and $R^{13}$ is a $C_{10}$ alkyl, $C_{12}$ alkyl, $C_{14}$ alkyl or $C_{16}$ alkyl group.

The metal hydroxide reactant is a mono-, bi-, or trivalent metal hydroxide, preferably a monovalent metal hydroxide, and most preferably an alkali metal hydroxide such as sodium hydroxide or potassium hydroxide. Special mention is made of potassium hydroxide. The metal chloride reaction product will precipitate and is easily removed, i.e. by filtration or the like, yielding a hydroxy quat/solvent reaction product. The hydroxy quat can be separated therefrom

by drying or the like.

The reaction is conducted in a solvent which comprises a $C_1$-$C_4$ normal alcohol. Preferably, the solvent is ethanol, and most preferably, anhydrous ethanol.

The amount of metal hydroxide reactant typically is a stoichiometric amount with respect to the quaternary ammonium chloride reactant. In practice, yield when using a stoichiometric amount of metal hydroxide reactant will range from about 65% to about 95%, but will vary, dependent in part upon the particular metal hydroxide reactant.

Yield can be further improved over conventional methods by utilization of a stoichiometric excess of metal hydroxide ranging from about 2% to about 20% excess. If an excess of metal hydroxide is used, yield will be increased to from about 95% to about 99%, again varying as above.

The unreacted metal hydroxide is soluble in the hydroxy quat/solvent mixture. Any excess or unreacted metal hydroxide should be removed after the reaction is completed, and is preferably precipitated by subsequent reaction with carbon dioxide to yield the corresponding metal carbonate. The carbonate is insoluble in the hydroxy quat/solvent mixture and is easily removed, i.e. by filtration or the like. Alternatively, a solid metal bicarbonate, in which the metal corresponds to the metal of the metal hydroxide, can be added and slurried with the hydroxy quat/solvent mixture. The soluble metal hydroxide reacts with solid bicarbonate to yield the insoluble metal carbonate. The metal carbonate does not react further with the hydroxy quat.

Mixing, adding, and reacting of the components in the preparation of these hydroxy quats can be accomplished by conventional means known to those of ordinary skill in the art. The order of addition of reactants or solvent does not affect the process. Reactants and/or solvent can be added sequentially or simultaneously in any suitable reaction vessel.

Typically, the reactants and solvent will be stirred and heated to from about 20°C to about 70°C and held at that temperature for a period of from about 1 hour to about 5 hours. The reaction mixture is then cooled, first to room temperature and then to about 0°C where it is held for about 1 hour to about 2 hours. Any precipitated metal chloride is collected as is known in the art, i.e. such as by filtration.

Alternatively, the reactants and solvent can be stirred at a slightly elevated temperature, i.e. from about 20°C to about 40°C, to yield the hydroxy quat/solvent mixture. Hydroxy quat can be separated as above.

<u>Quaternary Ammonium Carbonates</u>

Although any quaternary ammonium carbonates are suitable for use in the present invention, preferred carbonate quats have the formula

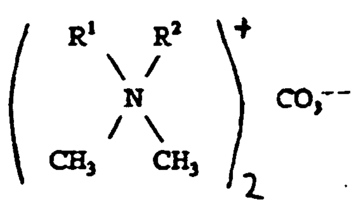

(XI)

wherein $R^1$ is a $C_1$-$C_{20}$ alkyl or aryl-substituted alkyl group, $R^2$ is a $C_8$-$C_{20}$ alkyl group, and preferably $R^1$ and $R^2$ are the same $C_8$-$C_{12}$ alkyl group.

Special mention is made of carbonate quats wherein $R^1$ is a methyl, $C_8$ alkyl, $C_9$ isoalkyl, $C_{10}$ alkyl, $C_{12}$ alkyl, $C_{14}$ alkyl, $C_{16}$ alkyl, or benzyl group; and $R^2$ is a $C_{10}$ alkyl, $C_{12}$ alkyl, $C_{14}$ alkyl, or $C_{16}$ alkyl group.

Most preferred carbonate quats are didecyldimethylammonium carbonate wherein $R^1$ and $R^2$ are a $C_{10}$ alkyl group and preferably an n-$C_{10}$ alkyl group. Didecyldimethylammonium carbonate, when observed as a 70-80 percent by weight solution is a yellow/orange liquid that has a slightly fruity odor. This formulation has a flash point of about 71°C (160 °F), and it reacts with carboxyl containing compounds.

One or more of these carbonate quats alone or in combination with the corresponding bicarbonate quat(s) and/or metal carbonate salt(s), preferably potassium carbonate salt, can be formulated in the present waterproofer, wood preservative systems.

The stability, and particularly the thermal stability, of carbonate quats is superior to that of hydroxy quats, making these carbonate quats suitable for concentrating and as stock intermediates for further processing.

Although certain carbonate quats can be prepared by a variety of methods, applicants have discovered an indirect synthesis method that can be used to prepare a variety of $C_1$-$C_{20}$ alkyl or aryl-substituted alkyl, $C_8$-$C_{20}$ alkyl quaternary ammonium carbonate compounds, preferably di $C_8$-$C_{12}$ alkyl quaternary ammonium carbonate compounds, and most preferably didecyldimethylammonium carbonate, according to the following schemes.

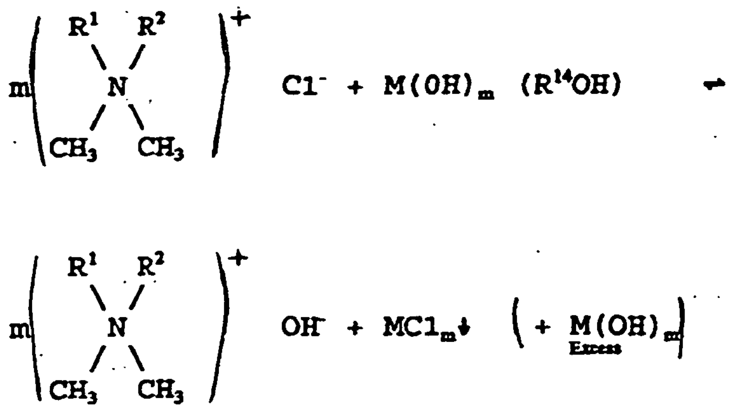

(XXVI)

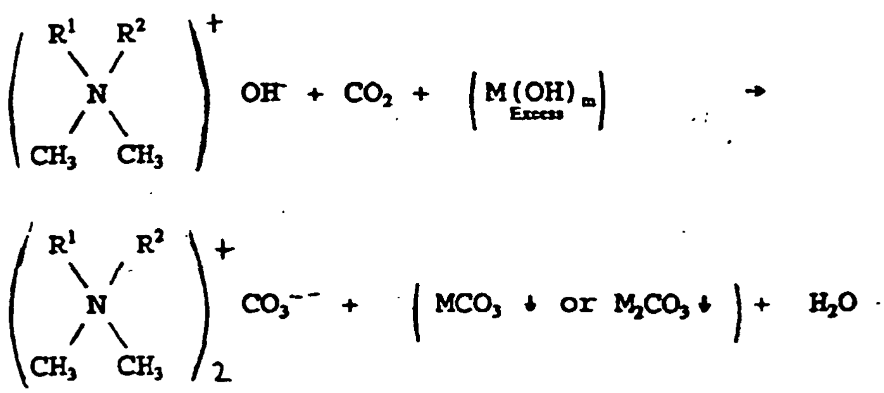

(XXVII)

wherein $R^1$ is a $C_1$-$C_{20}$ alkyl or aryl-substituted alkyl group; $R^2$ is a $C_8$-$C_{20}$ alkyl group; and preferably $R^1$ is the same as $R^2$ and $R^1$ is a $C_8$-$C_{12}$ alkyl group; $R^{14}$ is a straight chain $C_1$-$C_4$ alkyl group; M is a mono-, bi-, tri-valent metal, preferably a monovalent metal, and most preferably an alkali metal; and m is 1 if M is mono-valent, 2 if M is di-valent, and 3 if M is trivalent.

A $C_1$-$C_{20}$ alkyl or aryl-substituted alkyl, $C_8$-$C_{20}$ alkyl, and preferably a di $C_8$-$C_{12}$ alkyl, quaternary ammonium chloride is used as a starting material and is reacted with a metal hydroxide to yield a $C_1$-$C_{20}$ alkyl or aryl-substituted alkyl, $C_8$-$C_{20}$ alkyl, and preferably a di $C_8$-$C_{12}$ alkyl, quaternary ammonium hydroxide intermediate. The hydroxy quat intermediate (s) and any excess metal hydroxide are then reacted with carbon dioxide to yield the carbonate quat(s) and the metal carbonate.

Many di $C_8$- $C_{12}$ alkyl quaternary ammonium chlorides are suitable reactants to prepare the intermediate hydroxy quat and are described above. The selections of the $R^1$ and $R^2$ substituents of the chloride quat reactant are determinative of the hydroxy quat intermediate, and therefore, of the carbonate quat product.

The metal hydroxide reactant is also as described above.

The metal chloride first step reaction product will precipitate and is easily removed, i.e. by filtration or the like, yielding a hydroxy quat/solvent reaction product. The hydroxy quat can be separated therefrom by drying or the like, if desired.

The first reaction (XXVI) is conducted in a solvent as described above, and the amount of metal hydroxide reactant is as described above.

Hydroxy quat and any unreacted metal hydroxide are then reacted with at least a stoichiometric equivalent of carbon dioxide to yield the quaternary ammonium carbonate (s), and if any unreacted metal hydroxide is present, the metal carbonate (s). The conversion of the metal hydroxide to the metal carbonate is the preferred reaction of the two carbonations and will proceed more rapidly. The metal carbonate will precipitate and can be separated easily, i.e. by filtration or the like, leaving the stable carbonate quat (s) or carbonate quat (s) /solvent reaction product.

The carbonation step can also produce the bicarbonate quat or the metal carbonate quat as byproducts.

Mixing, adding, and reacting of the components in the preparation of these carbonate quats can be accomplished by conventional means known to those of ordinary skill in the art. The order of addition of reactants or solvent in any individual step does not affect the process. Reactants and/or solvent can be added sequentially or simultaneously in any suitable reaction vessel. For example, the metal hydroxide may be dissolved in alcohol and the resultant mixture

added to the chloride quat or the chloride quat may be dissolved in alcohol and the metal hydroxide added to the resultant mixture.

The carbon dioxide is generally bubbled for a suitable period known to those of ordinary skill in the art through the hydroxy quat/solvent supernatant after the metal chloride precipitate has been separated. Alternatively, the carbon dioxide can be added as solid dry ice directly to the hydroxy quat. Typically, this time varies from about 0.5 hour to about 1 hour at ambient temperature. Any precipitated metal carbonate is collected as is known in the art, i.e., such as by filtration.

<u>Quaternary Ammonium Carboxylates</u>

Although any quaternary ammonium carboxylates are suitable for use in the present invention, preferred carboxylate quats have the formula

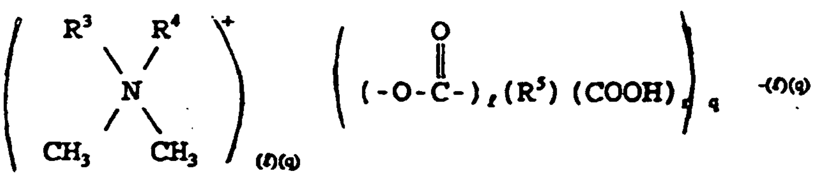 (XXVIII)

wherein $R^3$ is a $C_1$-$C_{20}$ alkyl or aryl-substituted alkyl group; $R^4$ is a $C_8$-$C_{20}$ alkyl group; but preferably $R^3$ and $R^4$ are the same $C_8$-$C_{12}$ alkyl group; $R^5$ is a substituted or unsubstituted, interrupted or uninterrupted $C_1$-$C_{100}$ group; $\ell$ and q independently are 1, 2, or 3, and ($\ell$) (q) is 1, 2, or 3; and r is 0 or an integer from 1 to 50.

Special mention is also made of carboxylate quats wherein $R^3$ is a methyl, $C_8$ alkyl, $C_9$ isoalkyl, $C_{10}$ alkyl, $C_{12}$ alkyl, $C_{14}$ alkyl or benzyl group; and $R^4$ is a $C_{10}$ alkyl, $C_{12}$ alkyl, $C_{14}$ alkyl or $C_{16}$ alkyl group. Most preferred carboxylate quats are didecyldimethylammonium carboxylates wherein $R^3$ and $R^4$ are a $C_{10}$ alkyl group and most preferably an n-$C_{10}$ alkyl group.

Preferred carboxyl anions are derived from saturated or unsaturated mono- or poly-, including, but not limited to, di- or tri-, carboxylic acids, and particularly $C_1$-$C_{20}$ carboxylic acids, or anhydrides thereof. $R^5$ independently can be substituted, particularly by one or more oxygen or boron atoms or sulfate groups, or interrupted, particularly by one or more oxygen or boron atoms or sulfate groups. Special mention is made of acetic acid, gluconic acid, lauric acid, formic acid, propionic acid, butyric acid, oxalic acid, acrylic acid; tartaric acid, benzoic acid, octanoic acid, and the like. Additionally, the carboxyl group can be derived from polymeric acids or copolymers in which one or more of the monomers is an acid. An example of a polyacid is polyacrylic acid. Examples of copolymer acids include, but are not limited to, olefin/carboxylic acid polymers such as poly(ethylene/acrylic acid).

Such acids, including the polymeric or copolymeric acids mentioned above are of the formula

$$((R^5)\ (COOH)_{(\ell+r)})_q \qquad \text{(XXIX)}$$

where $R^5$, $\ell$, r, and q are defined as above. In polymeric copolymers carboxylic acids, $R^5$ can be represented as $((R^{15})_s (R^{16})_t)$ giving

$$(R^{15})_s\ (R^{16})_t\ (COOH)_{(\ell+r)})_q \qquad \text{(XXX)}$$

where $R^{15}$ and $R^{16}$ independently are substituted or unsubstituted, interrupted or uninterrupted as above $C_1$-$C_{100}$ groups and s and t independently are integers from 1 to 100. Preferably, $R^5$, $R^{15}$, and $R^{16}$ independently are alkyl or alkenyl groups.

It is believed that the long chain carboxylate quat (s) enhance waterproofing properties of treated substrates.

Although the carboxylate quats can be prepared by a variety of methods, preferably they are prepared by an indirect synthesis, a direct synthesis, or a hydroxy quat/acid synthesis.

The indirect synthesis is illustrated below in schemes XXXI to XXXIII.

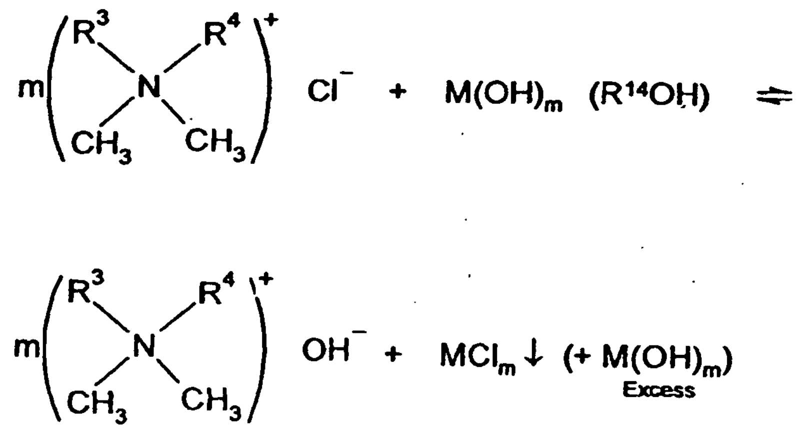


(XXXI)

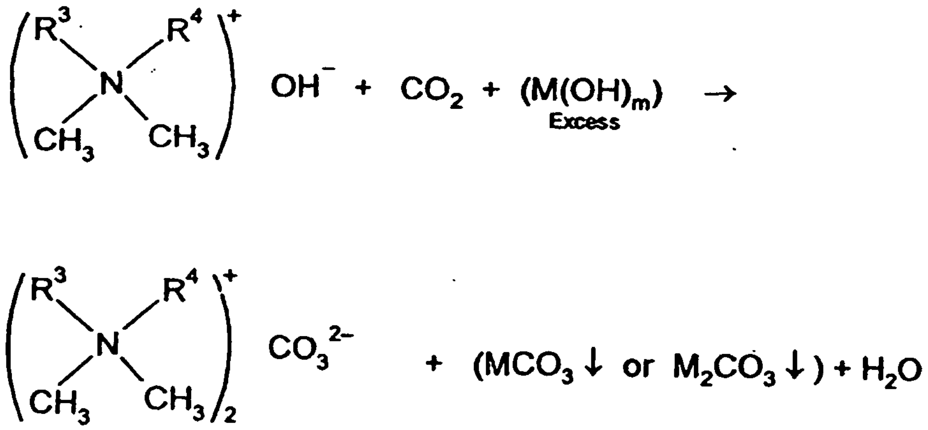


(XXXII)

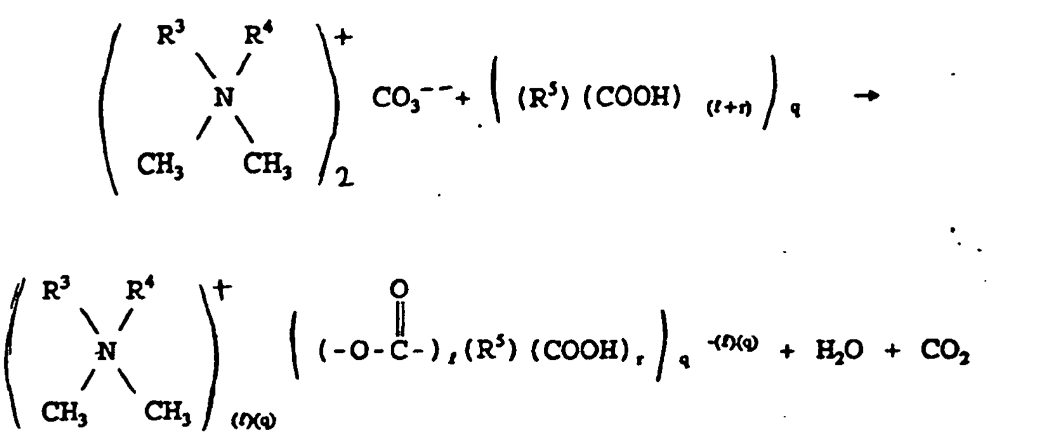

(XXXIII)

wherein $R^3$ is a $C_1$-$C_{20}$ alkyl or aryl-substituted alkyl group; $R^4$ is a $C_8$-$C_{20}$ alkyl group; and preferably $R^3$ is the same as $R^4$ and $R^4$ is a $C_8$-$C_{12}$ alkyl group; $R^{14}$ is a straight chain $C_1$-$C_4$ alkyl group; $R^5$ is a substituted or unsubstituted, as explained above, interrupted or uninterrupted, as explained above, $C_1$-$C_{100}$ group; $\ell$ and q independently are 1, 2, or 3 and (t) (q) is 1, 2, or 3; M is a mono-, bi-, tri-valent metal, preferably a monovalent metal, and most preferably an alkali metal; r is 0 or an integer from 1 to 50; and m is 1 if M is mono-valent, 2 if M is di-valent, and 3 if M is tri-valent.

The carboxylate quat is prepared via a carbonate quat intermediate.

A $C_1$-$C_{20}$ alkyl or aryl-substituted alkyl, $C_8$-$C_{20}$ alkyl, and preferably a di $C_8$-$C_{12}$ alkyl, quaternary ammonium chloride is used as a starting material and is reacted with a metal hydroxide to yield a $C_1$-$C_{20}$ alkyl or aryl-substituted alkyl, $C_8$-$C_{20}$ alkyl, and preferably a di $C_8$-$C_{12}$ alkyl, quaternary ammonium hydroxide intermediate as above. The

hydroxy quat intermediate(s) and any excess metal hydroxide are then reacted with carbon dioxide to yield the carbonate quat(s) and the metal carbonate(s) as above. The carbonate quat second intermediate(s) is then reacted with at least one carboxylic acid to yield the carboxylate quat (s) . The selection of the $C_8$-$C_{12}$ alkyl substituent of the chloride quat reactant is determinative of the hydroxy quat first intermediate, therefore, of the carbonate quat second intermediate, and ultimately, of the cation component of the carboxylate quat product.

The metal hydroxide reactant is described above. The preparation of the hydroxy quat is preferably conducted in a solvent as described above, and the amount of metal hydroxide reactant is described above.

Hydroxy quat and any unreacted metal hydroxide are then reacted with carbon dioxide to yield the quaternary ammonium carbonate(s) as detailed above. The carbonation step can also produce the bicarbonate quat(s) or the metal carbonate quat(s) as by-products.

The carbonate quat second intermediate(s) is then reacted with at least a stoichiometric amount of carboxylic acid(s) to yield the carboxylate quat(s).

The carboxylic acid(s) in reaction (XXXIII) is typically added over a short period of several minutes, and the reaction typically is rapid. The carboxylate quat (s) can be separated or concentrated by filtration or evaporation after a carbon dioxide evolution in this step is completed.

In the indirect synthesis, any acid having a pKa less than that of carbonic acid, i.e., less than 6.4, such as carboxylic, phosphoric, sulfonic acids, and the like, can be reacted with a carbonate quat and displace carbon dioxide.

Alternatively, the carboxylate quats can be prepared by a direct synthesis method. A metal salt of a carboxylic acid is reacted with a $C_1$-$C_{20}$ alkyl or aryl-substituted alkyl, $C_8$-$C_{20}$ alkyl, and preferably a di-$C_8$-$C_{12}$ alkyl, quaternary ammonium chloride, in a double replacement reaction, to yield the carboxylate quat and the metal chloride salt

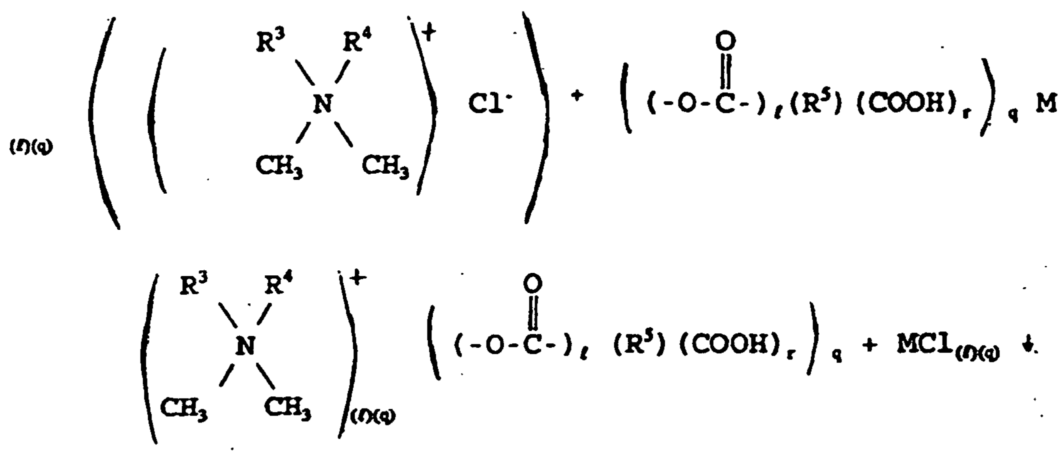

(XXXIV)

wherein $R^3$, $R^4$, $R^5$, M, $\ell$, q, and r are as defined above.

The metal carboxylates are derived from carboxylic acids. The carboxylic acids are as described and detailed above. The metals are mono-, di-, or the tri-valent metals, preferably mono-valent metals and most preferably alkali metals. Special mention is made of potassium and sodium.

Reaction (XXXIV) can be conducted neat or in a number of solvents including, but not limited to ethanol, acetic acid, or propionic acid. Preferably, the solvent comprises a $C_1$-$C_4$ normal alcohol as described above. Yield will depend on the solvent and the reaction conditions selected, which can be determined by one of ordinary skill in the art through routine experimentation in accordance with this detailed explanation.

The chloride quat starting material is selected as above, and again, its selection is determinative of the cation of the carboxylate quat to be formed.

Finally, a third method for the production of the carboxylate quat(s) includes reacting hydroxy quat (s) with carboxylic acid (s) as shown in scheme XXXV,

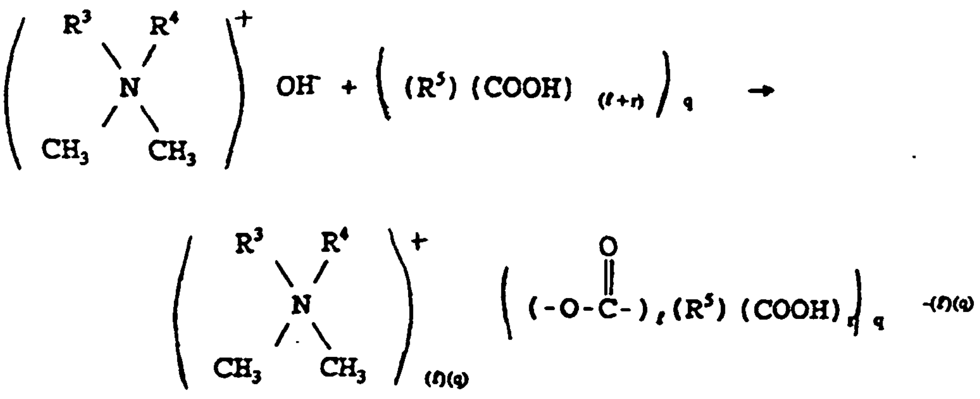

(XXXV)

wherein $R^3$, $R^4$, $R^5$, $\ell$, 1, and r are as defined above.

The hydroxy quat (s), carboxylic acid(s), and carboxylate quat(s) are as described above.

Mixing, adding, and reacting of the components in any of the direct, indirect or hydroxy quat/acid methods can be accomplished by conventional means known to those of ordinary skill in the art. The order of addition of reactants or solvent in any individual step does not affect the process.

Quaternary Ammonium Borates

Although any quaternary ammonium borates are suitable for use in the present invention, preferred borate quats have the formula

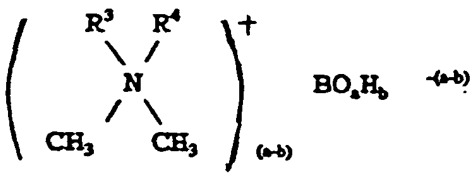

(XXXVI)

wherein $R^3$ is a $C_1$-$C_{20}$ alkyl or aryl-substituted alkyl group; $R^4$ is a $C_8$-$C_{20}$ alkyl group; but preferably $R^3$ and $R^4$ are the same $C_8$-$C_{12}$ alkyl group; a is 2 or 3, but when a is 2, b is 0 or 1 and when a is 3, b is 0, 1, or 2.

Special mention is also made of borate quats wherein $R^3$ is a methyl, $C_8$ alkyl, $C_9$ isoalkyl, $C_{10}$ alkyl, $C_{12}$ alkyl, $C_{14}$ alkyl or benzyl group; and $R^4$ is a $C_{10}$ alkyl, $C_{12}$ alkyl; $C_{14}$ alkyl or $C_{16}$ alkyl group. Most preferred borate quats are didecyldimethylammonium borates wherein $R^3$ and $R^4$ are a $C_{10}$ alkyl group and most preferably an n-$C_{10}$ alkyl group.

Typically; the production of the borate quat (s) includes reacting hydroxy quat(s) with boric acid.

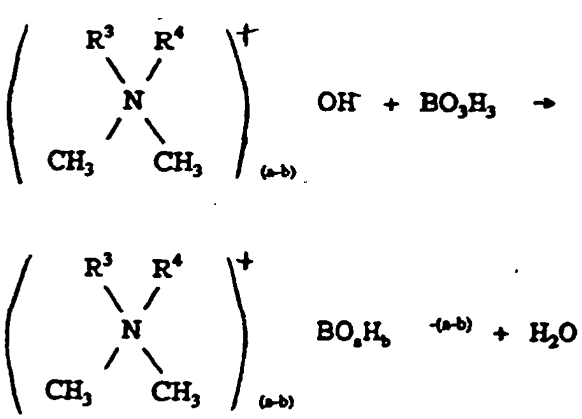

(XXXVII).

wherein $R^3$, $R^4$, a, and b are as defined above.

Mixing, adding, and reacting of the components in the hydroxy quat/acid method can be accomplished by conventional means known to those of ordinary skill in the art. The order of addition of reactants does not affect the process.

The following examples 1 to 38 illustrate the preparation of quaternary ammonium compounds which may be used in the invention, while examples 39 to 46 illustrate the invention without limitation. All parts and percentages are given by weight unless otherwise indicated.

Quaternary compounds are quantified by two phase titration with sodium laurylsulfate and an indicator. The mixture is buffered to a pH of 10.

Swell index is calculated as

$$\left( \frac{\text{(Swell of Control - Swell of Sample)}}{\text{Swell of Control}} \right) \times 100$$

PREPARATION OF HYDROXY QUATS

Example 1 - Stoichiometric Amount of Metal Hydroxide

180 g (0.4 mol) of 80% didecyldimethylammonium chloride in 20% ethanol water (144 g of DDAC), 180 ml of absolute denatured ethanol (denatured with methanol/isopropanol), and 26 g (0.4 mol) of 85% potassium hydroxide pellets (22.1 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.

Potassium chloride precipitated, and the precipitate was collected on a vacuum filter. The solid was washed with cold ethanol and subsequently was dried, yielding 30 g of dry potassium chloride. The quat solution was concentrated in a vacuum to about 75% active bases.

Yield was 180 g of product containing 138 g of didecyldimethylammonium hydroxide.

Example 2

The procedure of Example 1 was followed, but the mixture was stirred mechanically at 50°C for one hour. Potassium chloride precipitated, and the precipitate was collected on a vacuum filter. The solid was washed with cold ethanol and subsequently was dried, yielding 30 g of dry potassium chloride.

Yield was 180 g of product containing 138 g of didecyldimethylammonium hydroxide.

Example 3

0.022 mol of 85% potassium hydroxide pellets (1.23 g of KOH) was added to 0.022 mol of 80% didecyldimethylammonium chloride in 20% ethanol/water (8 g of DDAC) dissolved in 10 ml of ethanol. The resultant mixture was stirred and heated to 70°C and held at this temperature for one-half hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are summarized in Table 1.

Example 4

0.022 mol of 85% potassium hydroxide pellets (1.23 g of KOH) was added to 0.022 mol of 80% didecyldimethylammonium chloride in 20% ethanol/water (8 g of DDAC) dissolved in 10 ml of propanol. The resultant mixture was stirred and heated to 80°C and held at this temperature for one hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are illustrated in Table 1.

Example 5

The procedure of Example 3 was followed substituting sodium hydroxide for the potassium hydroxide.

Results are illustrated in Table 1.

Example 6

The procedure of Example 4 was followed substituting sodium hydroxide for the potassium hydroxide.
Results are illustrated in Table 1.

TABLE 1

| Preparation of Didecyldimethylammonium Hydroxide from Stoichiometric Amounts of Reactants | | | | |
|---|---|---|---|---|
| Example | 3 | 4 | 5 | 6 |
| Hydroxide | KOH | KOH | NaOH | NaOH |
| Solvent | Ethanol | n-propanol | Ethanol | n-propanol |
| Conversion % | 96 | 95 | 81 | 83 |

Examples 3-6 demonstrate that the use of a normal $C_1$-$C_4$ alcohol as a reaction medium enhances conversion of the chloride quat to the hydroxy quat. Furthermore, a comparison of examples 3 and 4 with Examples 5 and 6 illustrates the increase in conversion by the use of the preferred metal hydroxide, potassium hydroxide.

Stoichiometric Excess of Metal Hydroxide

Example 7

A nitrogen purged reactor equipped with a heating mantle and a magnetic stir bar was charged with 0.4 mol of 80% didecyldimethylammonium chloride (144 g of DDAC) in 20% ethanol/water, 180 ml of ethanol, and 0.49 mol of 85% potassium hydroxide (27.5 g of KOH) pellets. The mixture was heated at 60-70°C for 3 hours, allowed to cool to room temperature, and then cooled to 0°C for about one hour to precipitate potassium chloride. The precipitate was collected on a vacuum filter, and the solid was washed with cold ethanol. Potassium chloride yield was 30.8 g.
The supernatant solution, which contained the hydroxy quat and 0.09 mol of excess potassium hydroxide, was stirred with 2 g (0.045 mol) of carbon dioxide gas (from dry ice). The mixture was kept cold for an hour and then was vacuum filtered to remove 7.2 g (theoretical 6.2 g) of potassium carbonate.
Conversion percentage to the hydroxy quat was determined to be 99%.

Preparation of Carbonate Quats

Example 8

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.
Potassium chloride precipitated, and the precipitate was collected on a vacuum filter. The solid was washed with cold ethanol and subsequently was dried, yielding 31 g (calculated yield 29.6 g) of dry potassium chloride.
The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered to remove 7.2 g of potassium carbonate (6.2 g calculated), and the filtrate was concentrated to yield an orange/brown liquid with 80-85% carbonate quat in water/ethanol and less than 0.1% chloride quat having a product with 98 to 99% exchanged quat purity.

Example 9

180 g (0.4 mol) of 80% didecyldimethylammonium chloride in 20% ethanol water (144 g DDAC), 180 ml of absolute denatured ethanol (denatured with methanol/isopropanol), and 32 g (0.49 mol) of 85% potassium hydroxide pellets (27 g KOH) were mixed in a flask that was purged with nitrogen and equipped with a heating mantle and a magnetic stirrer. The mixture was heated to 50°C and stirred for one hour.
Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered, and the filtrate was concentrated to yield an orange/brown liquid. Yield was similar to that of Example 8.

Indirect Synthesis of Carboxylate Quat

Example 10 - Didecyldimethylammonium propionate

180 g (0.4 mol) of 80% didecyldimethylammonium chloride in 20% ethanol water (144 g of DDAC), 180 ml of absolute denatured ethanol (denatured with methanol/isopropanol), and 32 g (0.49 mol) of 85% potassium hydroxide pellets (27 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.

Potassium chloride precipitated, and the precipitate was collected on a vacuum filter. The solid was washed with cold ethanol and subsequently was dried, yielding 31 g (calculated yield 29.6 g) of dry potassium chloride.

The ethanolic solution of the hydroxy quat containing about 0.09 mol of unreacted KOH, was stirred while 50 g of carbon dioxide (from sublimed carbon dioxide) were bubbled over one half hour. The resultant mixture was then filtered to remove 7.2 g of potassium carbonate (6.2 g calculated), and the filtrate was concentrated to yield an orange/brown liquid with 80-85% carbonate quat (0.4 mol of carbonate quat) and less than 0.1% chloride for a product with 98 to 99% exchanged quat purity.

The cold product, after filtration, was placed in a closed flask equipped with a condenser, addition funnel, and a tube connected to a water displacement type gas measuring device. An equivalent (0.4 mol, 29.6 g), of propionic acid was added to the carbonate quat over five minutes. Immediate gas evolution was noted, and 5.75 l of gas were collected over 15 minutes. The solvent was removed on a rotary evaporator after the carbon dioxide evolution ceased, and yielded a yellow/orange liquid.

Quat analysis revealed that the product contained 85% active quat with 0.09% free chloride and 99% exchange.

Example 11 - Didecyldimethylammonium acetate

The procedure of Example 10 is followed, substituting 0.4 mol of acetic acid for the propionic acid.

Example 12 - Didecyldimethylammonium 2-ethylhexanoate

The procedure of Example 10 is followed, substituting 0.4 mol of 2-ethylhexanoic acid for the propionic acid.

The product is cloudy.

Example 13 - Didecyldimethylammonium gluconate

The procedure of Example 10 is followed, substituting 0.4 mol of gluconic acid for the propionic acid.

The product is water soluble.

Example 14 - Didecyldimethylammonium octanoate

The procedure of Example 10 is followed, substituting 0.4 mol of octanoic acid for the propionic acid.

Example 15 - Didecyldimethylammonium mixed coconut fatty acid carboxylate

The procedure of Example 10 is followed, substituting 0.4 mol of mixed coconut fatty acid for the propionic acid.

Example 16 - Didecyldimethylammonium laurate

The procedure of Example 10 is followed, substituting 0.4 mol of lauric acid for the propionic acid.

The product is a waxy solid.

Example 17 - Octyldecyldimethylammonium propionate

The procedure of Example 10 was followed, substituting 0.4 mol of 80% octyldecyldimethylammonium chloride

for the didecyldimethylammonium chloride to yield octyldecyldimethylammonium propionate.

Example 18 - Octyldecyldimethylammonium acetate

The procedure of Example 11 was followed, substituting 0.4 mol of 80% octyldecyldimethylammonium chloride for the didecyldimethylammonium chloride to yield octyldecyldimethylammonium acetate.

Example 19 - Isononyldecyldimethylammonium 2-ethylhexanoate

The procedure of Example 12 was followed, substituting 0.4 mol of 80% isononyldecyldimethylammonium chloride for the didecyldimethylammonium chloride to yield isononyldecyldimethylammonium 2-ethyl-hexanoate.

Example 20 - Isononyldecyldimethylammonium gluconate

The procedure of Example 13 was followed, substituting 0.4 mol of 80% isononyldecyldimethylammonium chloride for the didecyldimethylammonium chloride to yield isononyldecyldimethylammonium gluconate.

Example 21 - Benzyldodecyldimethylammonium gluconate

The procedure of Example 13 was followed, substituting 0.4 mol of 80% benzyldodecyldimethylammonium chloride for the didecyldimethylammonium chloride to yield benzyldodecyldimethylammonium gluconate.

Example 22 - Benzyldodecyldimethylammonium octanoate

The procedure of Example 14 was followed, substituting 0.4 mol of 80% benzyldodecyldimethylammonium chloride for the didecyldimethylammonium chloride to yield benzyldodecyldimethylammonium octanoate.

Example 23 - A mixture of benzyldodecyl-, benzyltetradecyl-, and benzylhexadecyldimethylammonium mixed coconut fatty acid carboxylate

The procedure of Example 15 was followed, substituting 0.4 mol of 80% of a mixture of benzyldodecyl-, benzyltetradecyl-, and benzylhexadecyldimethylammonium mixed fatty acid benzyldodecyldimethylammonium chloride for the didecyldimethylammonium chloride to yield a mixture of benzyldodecyl-, benzyltetradecyl-, and benzylhexadecyldimethylammonium mixed coconut fatty acid carboxylate.

Example 24 - A mixture of benzyldodecyl-, benzyltetradecyl-, and benzylhexadecyldimethylammonium laurate

The procedure of Example 16 was followed, substituting 0.4 mol of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium laurate.

Direct Synthesis of Carboxylate Quat

Example 25 - Didecyldimethylammonium acetate

180 g (0.4 mol) of 80% didecyldimethylammonium chloride in 20% ethanol water (144 g of DDAC), 180 ml of anhydrous ethanol, and a stoichiometric excess, 47 g (0.48 mol), of anhydrous potassium acetate was mixed in a flask that was purged with nitrogen and equipped with a heating mantle, a magnetic stirrer, and a condenser. The mixture was stirred and heated at 60-70°C for two hours. The insoluble potassium acetate crystals slowly dissolved and a finer solid (KCl) separated. The mixture was then cooled to 0°C and vacuum filtered. The solid washed with cold ethanol to remove 30.7 g of potassium chloride (theoretical 29.6 g). The solution was concentrated, cooled, and filtered to remove 6.5 g of potassium acetate (theoretical 29.6 g).

Additional fine crystals of potassium acetate settled out on standing. By assay, the light yellow liquid product was determined to be 80% quat with 100% exchange.

Example 26 - Didecyldimethylammonium gluconate

0.0221 mol of sodium gluconate and 0.0221 mol of 80% didecyldimethylammonium chloride in water were

mixed in a flask. The mixture was heated and held until evolution of carbon dioxide gas ceased.

The resultant quat was analyzed, and conversion was determined to be less than 20%.

Example 27 - Didecyldimethylammonium 2-ethylhexanoate

0.0221 mol of sodium 2-ethylhexanoate and 0.0221 mol of 80% didecyldimethylammonium chloride in water were mixed in a flask. The mixture was heated and held until evolution of carbon dioxide gas ceased.

The resultant quat was analyzed, and conversion was determined to be 77%.

Example 28 - Didecyldimethylammonium laurate

0.4 mol of sodium laurate and 0.4 mol of 80% didecyldimethylammonium chloride in water were mixed in a flask. The mixture was heated to 60°C and held for 1 hour.

The resultant quat was analyzed, and conversion was determined to be 90%

Example 29 - Didecyldimethylammonium propionate

0.0221 mol of sodium propionate and 0.0221 mol of 80% didecyldimethylammonium chloride in 8 g of propionic acid were mixed in a flask. The mixture was heated to 60°C-80°C and held for 2 hours.

The resultant quat was analyzed, and conversion was determined to be 90%

Example 30 - Didecyldimethylammonium propionate

0.4 mol of potassium propionate and 0.4 mol of 80% didecyldimethylammonium chloride in solid form were mixed in a flask. The mixture was heated to 60°C - 80°C and held for 2 hours.

The resultant quat was analyzed, and conversion was determined to be 91%

Hydroxy Quat/Acid Synthesis

Example 31 - Didecyldimethylammonium propionate

180 g (0.4 mol) of 80% didecyldimethylammonium chloride in 20% ethanol water (144 g of DDAC), 180 ml of absolute denatured ethanol (denatured with methanol/-isopropanol), and 26 g (0.4 mol) of 85% potassium hydroxide pellets (22 g of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.

Potassium chloride precipitated, and the precipitate was collected on a vacuum filter. The solid was washed with cold ethanol and subsequently was dried, yielding 30 g of dry potassium chloride.

The hydroxy quat/ethanol solution was mixed with a stoichiometric amount of propionic acid to yield a yellow/orange liquid having a flash point of 41.1 °C (106 °F).

Example 32 - Didecyldimethylammonium borate

The procedure of Example 31 is followed substituting 0.4 mol of boric acid for the propionic acid.

The product is a liquid.

Example 33 - Octyldecyldimethylammonium borate

The procedure of Example 32 was followed, substituting 0.4 mol of 80% octyldecyldimethylammonium chloride for the didecyldimethylammonium chloride to yield octyldecyldimethylammonium borate.

Example 34 Isononyldecyldimethylammonium borate

The procedure of Example 32 was followed, substituting 0.4 mol of 80% isononyldecyldimethylammonium chloride for the didecyldimethylammonium chloride to yield isononyldecyldimethylammonium borate.

Example 35 - Benzyldodecyldimethylammonium borate

The procedure of Example 32 was followed, substituting 0.4 mol of 80% benzyldodecyldimethylammonium chloride for the didecyldimethylammonium chloride to yield benzyldodecyldimethylammonium borate.

Example 36 - A mixture of benzyldodecyl-, benzyltetradecyl-, and benzylhexadecyldimethylammonium borate

The procedure of Example 32 was followed, substituting 0.4 mol of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium borate.

Example 37 - Dihexadecyldimethylammonium borate

The procedure of Example 32 was followed, substituting 0.4 mol of 80% dihexadecyldimethylammonium chloride for the didecyldimethylammonium chloride to yield dihexadecyldimethylammonium borate.

Example 38 - Dodecyltrimethylammonium borate

The procedure of Example 32 was followed, substituting 0.4 mol of 80% dodecyltrimethylammonium chloride for the didecyldimethylammonium chloride to yield dodecyltrimethylammonium borate.

Example 39 - DDAC/9% sorbitan tristearate (STS)/Water

3 parts of didecyldimethylammonium chloride and 9 parts of sorbitan tristearate are melted together and stirred while 88 parts of warm (40 °C) water are added to yield a stable emulsion which is suitable for waterproofing and preserving wood.

Example 40 - DDAC/9% sorbitan monostearate (SMS)/Water

The method of Example 39 is followed substituting 9 parts of SMS for the STS.

Example 41 - Didecyldimethylammonium carbonate/2.5% glycerol monolaurate (GML)/Water

The method of Example 39 is followed substituting 3 parts of didecyldimethylammonium carbonate for the didecyldimethylammonium chloride, 2.5 parts of GML for the STS, and 94.5 parts of water for the water.

Example 42 - Didecyldimethylammonium carbonate/2.5% glycerol monostearate (GMS)/Water

The method of Example 41 is followed substituting 2.5 parts of GMS for the GML.

Example 43 - Didecyldimethylammonium chloride/9% STS/Water

End grain pine wafers are weighed and then soaked with a waterproofer, wood preservative system prepared according to the method of Example 39 until the samples are saturated with the treating mixture. The samples are then air dried to constant weight to determine the uptake of the waterproofer, wood preservative system.
The treated wafers are removed, dried to constant weight, and weighed periodically to determine resistance to leaching.
The dried treated wafers are soaked in water for 30 minutes to determined dwelling. Swell is measured as the increase in length of the sample compared to an untreated control, and the swell index for each is calculated.
Results are illustrated in Table 2 and Figures 1A and 1B.

Comparative Example 43A - Didecyldimethylammonium chloride

The method of Example 43 is followed substituting didecyldimethylammonium chloride for the waterproofer, wood preservative system.
Results are illustrated in Table 2 and Figures 1A and 1B.

Example 44 - DDAC/9% SMS/Water

The method of Example 43 is followed substituting a waterproofer, wood preservative system prepared according to the method of Example 40 for the waterproofer, wood preservative system.
Results are illustrated in Table 2.

Comparative Example 44A - Didecyldimethylammonium hydroxide

The method of Comparative Example 43A is followed substituting didecyldimethylammonium hydroxide for the didecyldimethylammonium chloride.
Results are illustrated in Table 2 and Figures 1A and 1B.

Comparative Example 44B - Didecyldimethylammonium carbonate

The method of Comparative Example 43A is followed substituting didecyldimethylammonium carbonate for the didecyldimethylammonium chloride to yield a clear solution.
Results are illustrated in Table 2 and Figures 1A and 1B.

Example 45 - Didecyldimethylammonium carbonate/2.5% GML/Water

The method of Example 43 is followed substituting 2.5 parts of GML for the STS, and 94.5 parts of water for the water.
Results are illustrated in Table 2.

Example 46 - Didecyldimethylammonium carbonate/2.5% GMS/Water

The method of Example 43 is followed substituting 2.5 parts of GMS for the STS, and 94.5 parts of water for the water.
Results are illustrated in Table 2.

Comparative Example 46A - Didecyldimethylammonium acetate

The method of Comparative Example 43A is followed substituting didecyldimethylammonium acetate for the didecyldimethylammonium chloride.
Results are illustrated in Figures 1A and 1B.

Comparative Example 46B - Didecyldimethylammonium mixed coconut fatty acid carboxylate

The method of Comparative Example 43A is followed substituting 5 parts of didecyldimethylammonium mixed coconut fatty acid carboxylate and 95 parts of water for the waterproofer, wood preservative system.
Results are illustrated in Table 2.

Comparative Example 46C - Mineral spirits

The method of Example 43 is followed substituting a commercially available wax based biocide/mineral spirit based solution (Woodtreat MB® - KopCoat, Inc.) for the waterproofer, wood preservative system.2
Results are illustrated in Table 2.
Table 2 illustrates the enhanced properties of waterproofer, wood preservative systems of the present invention.

Table 2

| Example | 43A | 43 | 44 | 44A | 44B | 45 | 46 | 46A | 46B | 46C |
|---|---|---|---|---|---|---|---|---|---|---|
| Composition | | | | | | | | | | |
| Quat | | | | | | | | | | |
| Chloride | 100 | 3 | 3 | - | - | - | - | - | - | - |

Table 2 (continued)

| Example | 43A | 43 | 44 | 44A | 44B | 45 | 46 | 46A | 46B | 46C |
|---|---|---|---|---|---|---|---|---|---|---|
| Composition | | | | | | | | | | |
| Quat | | | | | | | | | | |
| Hydroxy | - | - | - | 100 | - | - | - | - | - | - |
| Carbonate | - | - | - | - | 100 | 3 | 3 | - | - | - |
| Acetate | - | - | - | - | - | - | - | 100 | - | - |
| Mixed Coconut Fatty Acid Carboxylate | - | - | - | - | - | - | - | - | 5 | - |
| Waterproofer | | | | | | | | | | |
| STS | - | 9 | - | - | - | - | - | - | - | - |
| SMS | - | - | 9 | - | - | - | - | - | - | - |
| GML | - | - | - | - | - | 2.5 | - | - | - | - |
| GMS | - | - | - | - | - | - | 2.5 | - | - | - |
| Wax-Based in Mineral Spirits | - | - | - | - | - | - | - | - | - | 1-2 |
| Solvent | | | | | | | | | | |
| Water | - | 88 | 88 | - | - | 94.5 | 94.5 | - | 95 | - |
| Mineral Spirits | - | - | - | - | - | - | - | - | - | 98-99 |
| Properties | | | | | | | | | | |
| Swell index (%) | - | 9 | 64 | - | - | 14 | 57 | - | 14 | 43 |
| Total Add On(%) | 35 | - | - | 35 | 37 | 4.2 | 5.1 | 45 | 2.7 | 3.4 |
| Solids or Add On Retained at 24 Hours Leaching, at Room Temperature (%) | - | - | - | - | - | - | - | - | 103 | 100+ |
| Solids or Add On Retained at 300 Hours Leaching, at Room Temperature (%) | 0 | 0 | - | 0 | 0 | 2.1 | 3.4 | 0 | - | - |

## Claims

1. The use of a waterproofer composition for the treatment and preservation of wood wherein said waterproofer composition has the formula

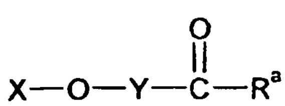

wherein:

X is hydrogen or

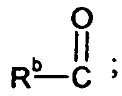

Y is

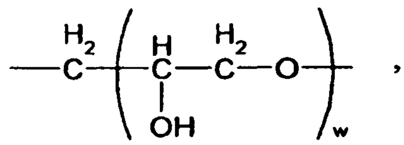

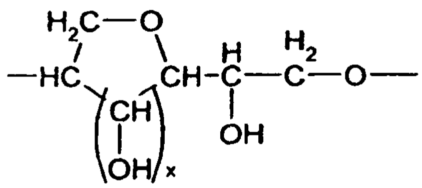

or an enantiomer thereof, or

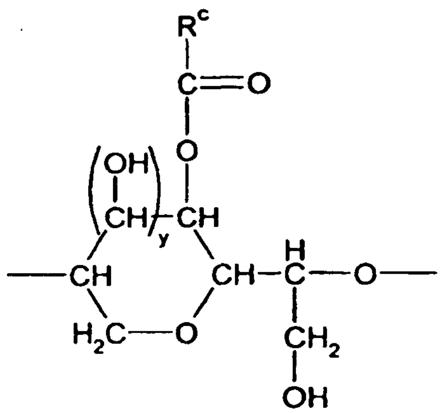

or an enantiomer thereof;
$R^a$, $R^b$ and $R^c$ independently are a saturated or unsaturated $C_9$-$C_{50}$ group;
w is an integer from 1 to 10; and
x is 1 and y is 0;
said composition containing a biocidal effective amount of at least one $C_1$-$C_{20}$ alkyl or aryl-substituted alkyl, $C_8$-$C_{20}$ alkyl quaternary ammonium composition and a solvent,
wherein said waterproofer composition is metal-free.

**2.** The use of a waterproofer composition for the treatment and preservation of wood as defined in claim 1 wherein:

X is hydrogen;
Y is

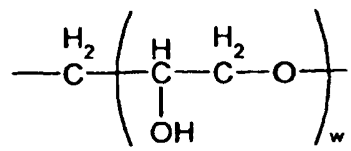

$R^a$ is a $C_{11}$-$C_{17}$ alkyl group, and
w is 1.

**3.** The use of a waterproofer composition for the treatment and preservation of wood as defined in claim 2 wherein:

$R^a$ is a $C_{11}$ alkyl group.

**4.** The use of a waterproofer composition for the treatment and preservation of wood as defined in claim 1 wherein:

X is hydrogen;

Y is

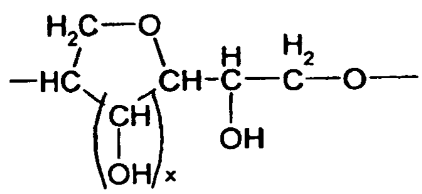

or an enantiomer thereof;

and $R^a$ is a $C_{17}$ alkyl group.

**5.** The use of a waterproofer composition for the treatment and preservation of wood as defined in claim 1 wherein:

X is

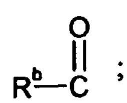

Y is

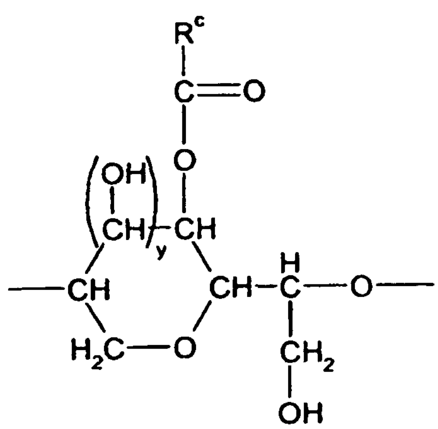

or an enantiomer thereof;

and $R^a$, $R^b$ and $R^c$ each are a $C_{17}$ alkyl group.

## Patentansprüche

1. Verwendung einer Hydrophobierungsmittelzusammensetzung für die Behandlung und Konservierung von Holz, worin die Hydrophobierungsmittelzusammensetzung die Formel

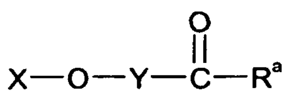

hat, worin

X Wasserstoff oder

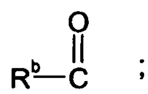

Y

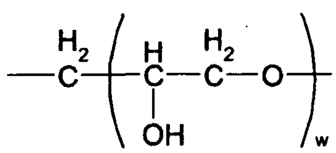

,

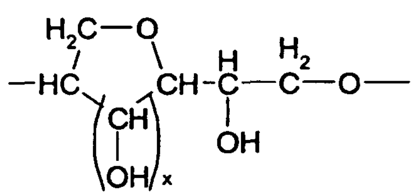

oder ein Enantiomeres davon, oder

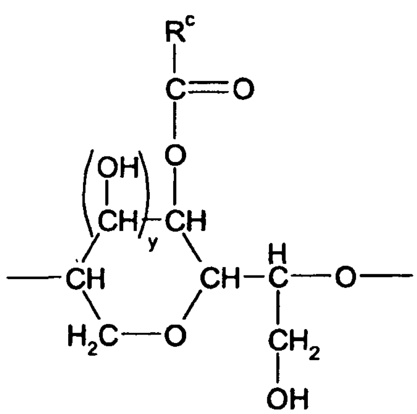

oder ein Enantiomeres davon ist;
$R^a$, $R^b$ und $R^c$ jeweils eine gesättigte oder ungesättigte $C_9$-$C_{50}$-Gruppe sind;
w eine ganze Zahl von 1 bis 10 ist;
und x eins und y null ist;
besagte Zusammensetzung eine biozid wirksame Menge wenigstens einer quartären $C_1$-$C_{20}$-Alkyl- oder (arylsubstituiertes Alkyl)-$C_8$-$C_{20}$-alkylammoniumverbindung und ein Lösungsmittel enthält, und worin besagte Hydrophobierungsmittelzusammensetzung metallfrei ist.

**2.** Verwendung einer Hydrophobierungsmittelzusammensetzung für die Behandlung und Konservierung von Holz; wie in Anspruch 1 definiert, worin:

X Wasserstoff,

Y

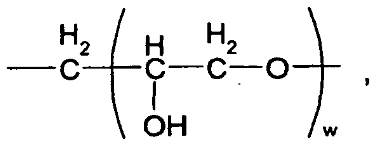

$R^a$ eine $C_{11}$-$C_{17}$-Alkylgruppe und w eins ist.

**3.** Verwendung einer Hydrophobierungsmittelzusammensetzung für die Behandlung und Konservierung von Holz, wie in Anspruch 2 definiert, worin $R^a$ eine $C_{11}$-Alkylgruppe ist.

**4.** Verwendung einer Hydrophobierungsmittelzusammensetzung für die Behandlung und Konservierung von Holz, wie in Anspruch 1 definiert, worin:

X Wasserstoff,

Y

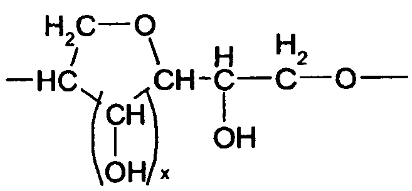

oder ein Enantiomeres davon,

und $R^a$ eine $C_{17}$-Alkylgruppe ist.

**5.** Verwendung einer Hydrophobierungsmittelzusammensetzung für die Behandlung und Konservierung von Holz, wie in Anspruch 1 definiert, worin

X

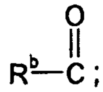

und Y

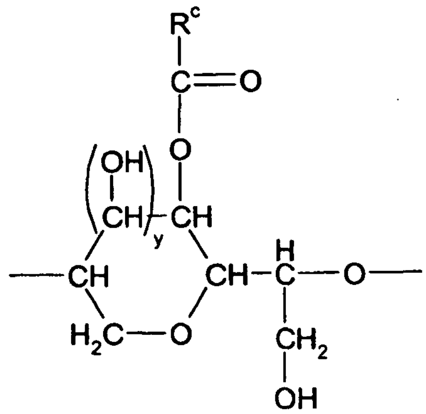

oder ein Enantiomeres davon ist,

und $R^a$, $R^b$ et $R^c$ jeweils eine $C_{17}$-Alkylgruppe sind.

## Revendications

**1.** Utilisation d'une composition hydrophobisante pour le traitement et la préservation du bois, dans laquelle ladite composition hydrophobisante a la formule

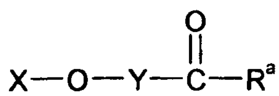

où :

X est l'hydrogène ou

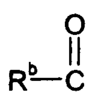 ;

Y est

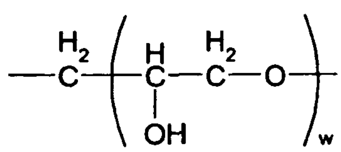 ,

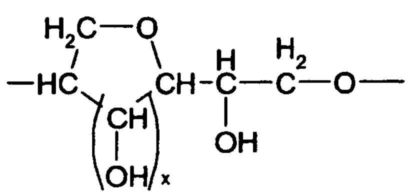

ou un énantiomère de celui-ci, ou

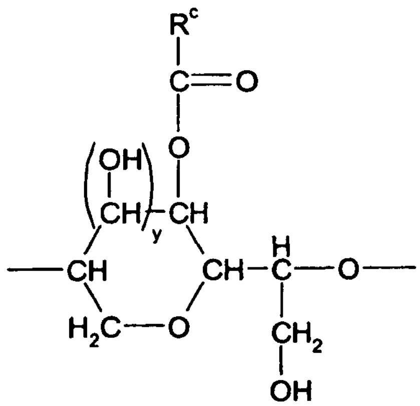

ou un énantiomère de celui-ci ;
$R^a$, $R^b$ et $R^c$ représentent, indépendamment les uns des autres, des groupements $C_9$-$C_{50}$ saturés ou insaturés ;
w est un nombre entier de 1 à 10 ; et
x est 1 et y est 0 ;
ladite composition contenant une quantité efficace d'un biocide d'au moins une composition d'ammonium quaternaire d'alkyle en $C_1$-$C_{20}$ ou d'alkyle en $C_1$-$C_{20}$ substitué par un aryle et d'alkyle en $C_8$-$C_{20}$ et un solvant, dans laquelle ladite composition ne contient aucun métal.

**2.** Utilisation d'une composition hydrophobisante pour le traitement et la préservation du bois comme définie dans la revendication 1, où :

X est un hydrogène,

Y est

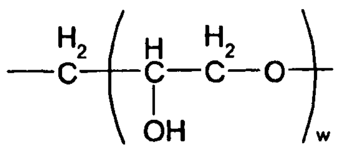,

$R^a$ est un groupement alkyle en $C_{11}$-$C_{17}$ et w est 1.

**3.** Utilisation d'une composition hydrophobisante pour le traitement et la préservation du bois comme définie dans la revendication 2, où :

$R^a$ est un groupement alkyle en $C_{11}$.

**4.** Utilisation d'une composition hydrophobisante pour le traitement et la préservation du bois comme définie dans la revendication 1, où :

X est un hydrogène ;

Y est

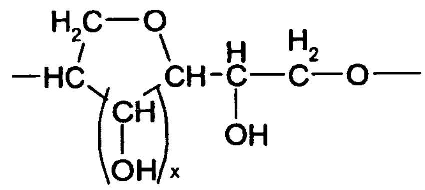

ou un énantiomère de celui-ci ;

et $R^a$ est un groupement alkyle en $C_{17}$.

**5.** Utilisation d'une composition hydrophobisante pour le traitement et la préservation du bois comme définie dans la revendication 1, où :

X est

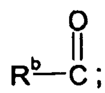

Y est

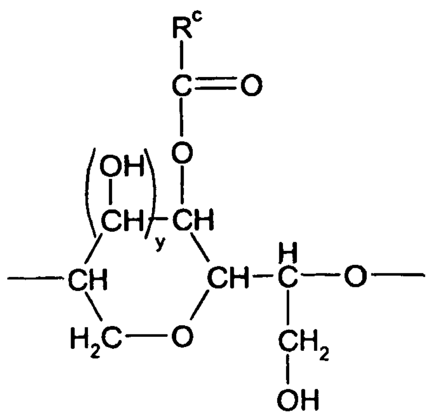

ou un énantiomère de celui-ci ;

et $R^a$, $R^b$ et $R^c$ représentent chacun un groupement alkyle en $C_{17}$.

FIG. 1A

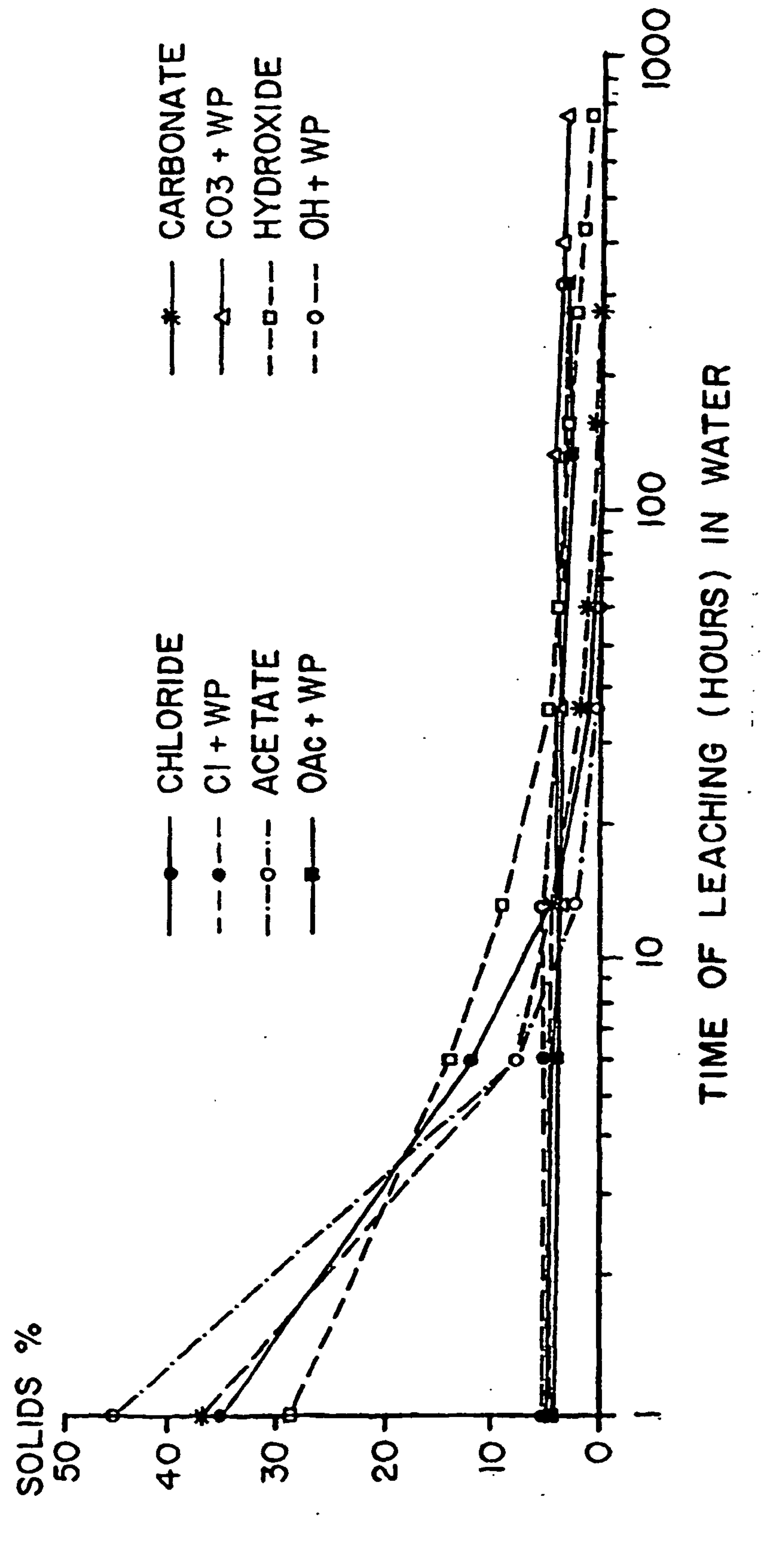
CHLORIDE
Cl + WP
ACETATE
OAc + WP
CARBONATE
CO3 + WP
HYDROXIDE
OH + WP
SOLIDS %
50
40
30
20
10
0
1
10
100
1000
TIME OF LEACHING (HOURS) IN WATER

FIG. 1B

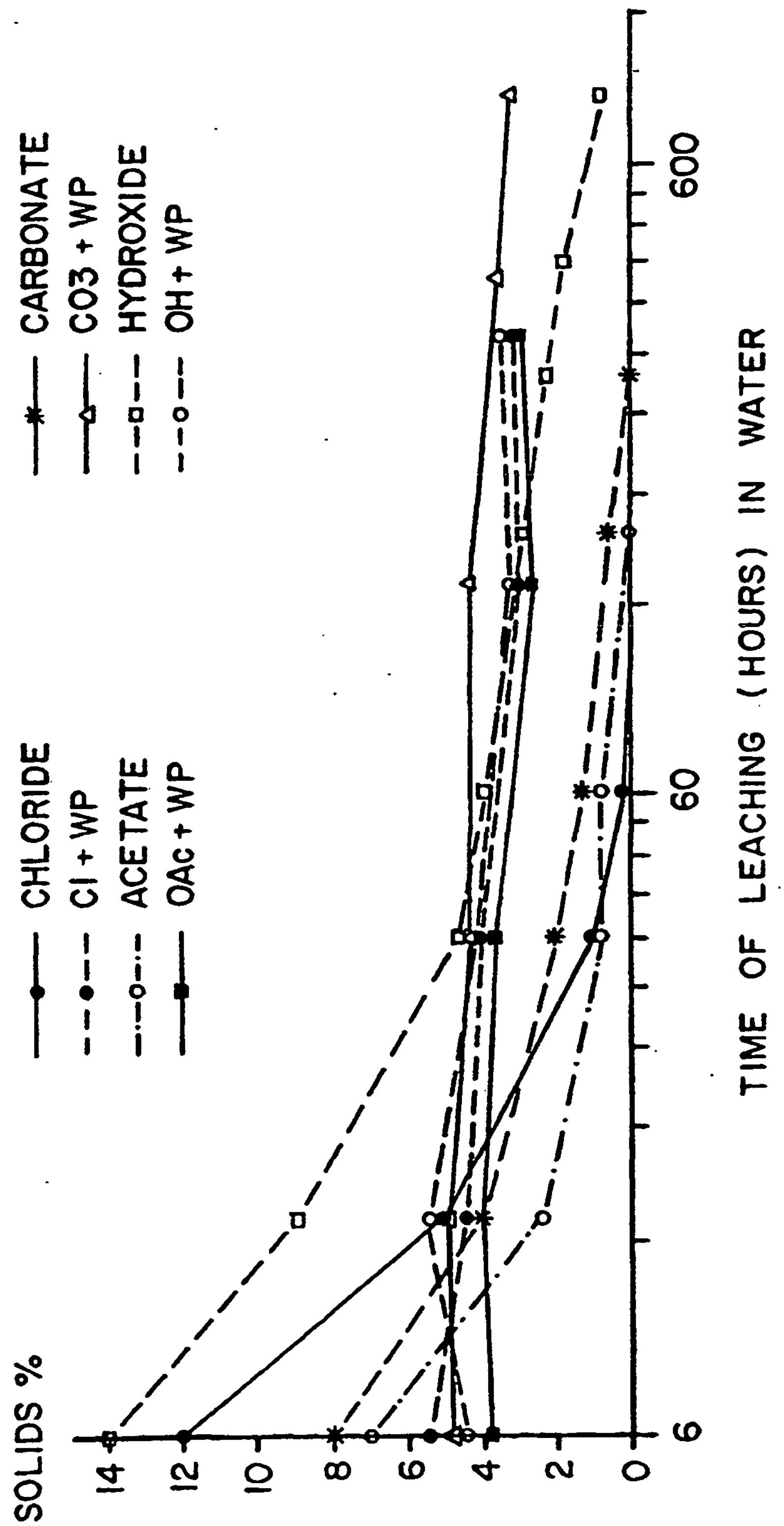
SOLIDS %
14
12
10
8
6
4
2
0
6
60
600
TIME OF LEACHING (HOURS) IN WATER
CHLORIDE
Cl + WP
ACETATE
OAc + WP
CARBONATE
CO3 + WP
HYDROXIDE
OH + WP